# EUROPEAN PATENT APPLICATION

(11) **EP 4 644 531 A1**
(43) Date of publication of application: **05.11.2025**
(21) Application number: 24818563.9
(22) Date of filing: 31.05.2024
(51) Int. Cl.: C12N 1/20, A61K 35/741, A61K 35/744, A61K 35/745, A61P 1/00, A61P 31/04, A61P 35/00

(54) **PROBIOTIC COMPOSITION FOR PREVENTING AND TREATING ANTI-TUMOR TREATMENT-RELATED DIARRHEA AND USE THEREOF**

(30) Priority: 06.06.2023 CN 202310664382
(71) Applicant: Sichuan Anaerobic Biotechnology Co., Ltd., Chengdu, Sichuan 610219 (CN)
(72) Inventor: CHENG, Lei, Chengdu, Sichuan 610219 (CN); LI, Jingxin, Chengdu, Sichuan 610219 (CN); ZHANG, Xufei, Chengdu, Sichuan 610219 (CN); LAN, Yan, Chengdu, Sichuan 610219 (CN); GAO, Xiang, Chengdu, Sichuan 610219 (CN)
(74) Representative: karo IP
(86) International application number: PCT/CN2024/096562
(87) International publication number: WO 2024/251031

(57) **Abstract**

The present disclosure relates to the technical field of microorganisms. The present disclosure relates to a probiotic composition and use thereof in the preparation of a product for preventing and/or treating health conditions such as cancer treatment-related diarrhea. The active ingredients of the probiotic composition include *Bifidobacterium bifidum, Enterococcus avium, Lactobacillus salivarius, Limosilactobacillus fermentum, and Parabacteroides distasonis.* The probiotic composition has good safety, auto-aggregation ability, antioxidant capacity, ability to produce short-chain fatty acids, *in vitro* cell adhesion ability, ability to inhibit multiple pathogenic bacteria, ability to treat cancer treatment-related diarrhea, etc.

## Description

### TECHNICAL FIELD

The present disclosure pertains to the field of microbial drugs, and relates to a probiotic composition and use thereof, and particularly to a composition comprising five probiotics and use thereof in the prevention and/or treatment of cancer treatment-related diarrhea.

### BACKGROUND

Cancer treatment-related diarrhea/tumor-associated diarrhea is a common complication of various anti-tumor adjuvant therapies and is one of the symptoms with the most significant impact on health and quality of life. In severe cases, it may also lead to treatment delays and reduced compliance, all of which may affect long-term outcomes of tumor treatment and are potentially fatal. Data released by the International Agency for Research on Cancer under the World Health Organization show that there were about 19.3 million new cancer cases worldwide in 2020, and it is predicted that the number of new cancer cases worldwide will reach 28.4 million by 2040. Up to 82% of patients receiving cancer treatment experience side effects such as diarrhea.

Chemotherapy (particularly chemotherapy using traditional chemotherapeutic drugs such as fluoropyrimidine drugs and irinotecan (CPT-11) which are the first-line drugs for treating metastatic colorectal cancer patients), radiotherapy, targeted therapy, and immunotherapy are major contributors to cancer treatment-related diarrhea aside from surgery. The overall incidence of diarrhea caused by treatment with fluoropyrimidine drugs and irinotecan is 50%-80%, with the incidence of severe (grade 3-5) diarrhea reaching 30% or more. Among these cases, 1%-5% of subjects receiving 5-fluorouracil (5-FU) died due to diarrhea. About 70% of patients with abdominal or pelvic tumors require radiotherapy. Among them, 60%-70% develop acute symptoms such as abdominal pain, diarrhea, and hematochezia, while 30%-40% develop chronic diarrhea. The incidence of diarrhea caused by targeted therapy (e.g., targeted therapies using multiple tyrosine kinase inhibitors (TKIs) such as afatinib, ceritinib, erlotinib, and lapatinib) may be up to 90% or more. Immune checkpoint inhibitors often cause immune-related enteritis. When used to treat tumors, ipilimumab, nivolumab, and pembrolizumab cause diarrhea in 1%-45% of tumor patients.

There exists commonality in the pathogenesis of diarrhea related to cancer treatments other than surgery. Cancer treatment-related diarrhea is caused by a combination of multiple factors, with the primary pathogenic factors including the following: 1) chemotherapeutic drugs and ionizing radiation during radiotherapy result in oxidative stress and DNA damage; 2) intestinal mucosal damage causes loss of epithelial cells, decreased integrity of tight junctions, death of crypt epithelial cells, and increased intestinal permeability; 3) various signaling pathways such as the nuclear factor NF-κB are highly activated, stimulating cells to produce inflammatory factors and thus causing mucosal inflammation; 4) damage to the small intestinal mucosa leads to bile acid malabsorption, and excessive bile acids (especially dihydroxy components) induce secretion of water and electrolytes and increase intestinal peristalsis, resulting in diarrhea; 5) infections by common nosocomial pathogens (*Salmonella, Shigella, Escherichia coli, Clostridioides difficile,* etc.); 6) bacterial translocation and intestinal dysbacteriosis, characterized by reduced probiotics and increased pathogenic bacteria; and 7) immune abnormalities, such as general activation of T cells.

According to current international clinical guidelines such as "Guidance on the management of diarrhoea during cancer chemotherapy (2014)" and "Diarrhoea in adult cancer patients: ESMO Clinical Practice Guidelines (2018)", the initial therapeutic drug recommended for chemotherapy-induced diarrhea (CID), radiation enteritis, targeted therapy-related diarrhea, and immune checkpoint inhibitor-related diarrhea is loperamide. The guidelines recommend octreotide for patients unresponsive to loperamide treatment. Loperamide administration should not be performed for more than 48 h. High-dose loperamide use may also carry risks of paralytic ileus and severe cardiotoxicity. Octreotide (first-line medication for patients with grade 3 or higher diarrhea scores) may cause side effects such as gallstones, hyperglycemia, and impaired glucose tolerance. The guidelines also recommend budesonide as a second-line therapeutic drug when loperamide treatment fails. However, glucocorticoids may exert systemic effects, causing an increased risk of infection and further exacerbation of infections such as viral and bacterial infections. For patients with grade 3-4 diarrhea complicated by neutropenia, oral antibiotics can be administered. However, antibiotics may cause diarrhea exacerbation and increase the risk of *Clostridioides difficile* infection. Tumor treatment often involves combination therapy employing multiple therapeutic modalities. The combination therapy greatly increases the incidence of diarrhea. If diarrhea persists for a long time or effective treatment is not taken after diarrhea onset, patients may develop symptoms such as electrolyte disturbances, dehydration, and anemia. Moderate or severe diarrhea may also lead to serious complications manifested as neutropenia and systemic inflammatory response syndrome. Therefore, there is an urgent clinical need for a new generation of drugs that are safer and more effective for cancer treatment-related diarrhea.

Intestinal probiotics are a type of active microorganisms that colonize the human intestinal tract and change the composition of the host's intestinal flora, conferring beneficial effects to the host. The intestinal probiotics offer advantages such as high safety, minimal toxic and side effects, broad indications, and extensive population coverage. The probiotic composition can exert a therapeutic effect on the complex pathogenesis of cancer treatment-related diarrhea, mainly reflected in that: 1) the probiotics achieve an antioxidant function by chelating metal ions, up-regulating their own and host antioxidant enzymes and metabolites, reducing the activity of ROS-producing enzymes, and the like; 2) the probiotics can produce beneficial secondary metabolites, such as short-chain fatty acids, indole derivatives, and secondary bile acids (the beneficial secondary metabolites enhance intestinal barrier function by stimulating goblet cells to secrete mucins, promoting epidermal cell repair, inhibiting apoptosis, enhancing tight junction proteins, etc.); 3) the probiotics regulate immune responses by down-regulating the NF-κβ pathway, inhibiting pro-inflammatory factors, secreting anti-inflammatory factors, and the like, thereby playing a role in resisting inflammation and enhancing body immunity; 4) the probiotics regulate intestinal flora balance, reduce pathogenic bacteria, and increase probiotics, thereby maintaining a normal intestinal homeostasis environment; 5) the probiotics regulate bile acid metabolism by increasing bile salt hydrolase activity in flora, inhibiting the FXR-FGF15 signaling pathway, and the like, thereby alleviating bile acid diarrhea; and 6) the probiotics inhibit the growth of pathogenic bacteria by increasing secretion of antibacterial proteins (defensins), producing antibacterial substances such as bacteriocins, competing with pathogens for adhesion sites, and the like.

The Chinese patent CN112694992B discloses a *Bifidobacterium bifidum* strain, which can alleviate diarrhea caused by enterotoxigenic *Escherichia coli* (ETEC). The Chinese patent CN113234619B discloses a *Bifidobacterium bifidum* strain capable of alleviating acute intestinal injury. A small number of literature reports that *Parabacteroides distasonis* has the effect of alleviating intestinal inflammation. For example, M. Kverka et al. (Oral administration of Parabacteroides distasonis antigens attenuates experimental murine colitis through modulation of immunity and microbiota composition) reported that *Parabacteroides distasonis* is able to ameliorate DSS-induced colitis in mice. At present, only a few documents report the use of *Lactobacillus salivarius* in the prevention and treatment of diarrhea. For example, the patent CN110878267B discloses a Lactobacillus salivarius strain ZLp4b, which can significantly mitigate and cure diarrhea in young stock. In addition to the patents and literature described above, there are some documents on the use of similar probiotics in the prevention and treatment of diarrhea or intestinal inflammation. However, current probiotic formulations are primarily used for treating common diarrhea symptoms and are mostly single strains, making it difficult to exert a targeted therapeutic effect on the complex pathogenesis of cancer treatment-related diarrhea.

### SUMMARY

The first aspect of the present disclosure provides a probiotic composition, which comprises any three, any four, or five of a first probiotic, a second probiotic, a third probiotic, a fourth probiotic, and a fifth probiotic (preferably, the probiotic composition comprises the fifth probiotic and any two, any three, or four of the first probiotic, the second probiotic, the third probiotic, and the fourth probiotic, and more preferably, the probiotic composition comprises the fourth probiotic, the fifth probiotic, and any two or any three of the first probiotic, the second probiotic, and the third probiotic), wherein the first probiotic is selected from *Bifidobacterium bifidum,* a progeny strain of the *Bifidobacterium bifidum,* a clonal strain of the *Bifidobacterium bifidum,* and a pure culture of the *Bifidobacterium bifidum*; the second probiotic is selected from *Enterococcus avium,* a progeny strain of the *Enterococcus avium,* a clonal strain of the *Enterococcus avium,* and a pure culture of the *Enterococcus avium*; the third probiotic is selected from *Lactobacillus salivarius,* a progeny strain of the *Lactobacillus salivarius,* a clonal strain of the *Lactobacillus salivarius,* and a pure culture of the *Lactobacillus salivarius*; the fourth probiotic is selected from *Limosilactobacillus fermentum,* a progeny strain of the *Limosilactobacillus fermentum,* a clonal strain of the *Limosilactobacillus fermentum,* and a pure culture of the *Limosilactobacillus fermentum*; and the fifth probiotic is selected from *Parabacteroides distasonis,* a progeny strain of the *Parabacteroides distasonis,* a clonal strain of the *Parabacteroides distasonis,* and a pure culture of the *Parabacteroides distasonis.*

In some embodiments, the *Bifidobacterium bifidum* mentioned above is the *Bifidobacterium bifidum* with a microbial deposit number of CCTCC NO: M2023349; the *Enterococcus avium* mentioned above is the *Enterococcus avium* with a microbial deposit number of CCTCC NO: M2023350; the *Lactobacillus salivarius* mentioned above is the *Lactobacillus salivarius* with a microbial deposit number of CCTCC NO: M2023348; the *Limosilactobacillus fermentum* mentioned above is the *Limosilactobacillus fermentum* with a microbial deposit number of CCTCC NO: M2023352; and the *Parabacteroides distasonis* mentioned above is the *Parabacteroides distasonis* with a microbial deposit number of CCTCC NO: M20222033.

In some embodiments, the probiotic composition comprises a first probiotic, a second probiotic, a third probiotic, a fourth probiotic, and a fifth probiotic, wherein the first probiotic is selected from *Bifidobacterium bifidum* with a microbial deposit number of CCTCC NO: M2023349, a progeny strain of the *Bifidobacterium bifidum* with the microbial deposit number of CCTCC NO: M2023349, a clonal strain of the *Bifidobacterium bifidum* with the microbial deposit number of CCTCC NO: M2023349, and a pure culture of the *Bifidobacterium bifidum* with the microbial deposit number of CCTCC NO: M2023349; the second probiotic is selected from *Enterococcus avium* with a microbial deposit number of CCTCC NO: M2023350, a progeny strain of the *Enterococcus avium* with the microbial deposit number of CCTCC NO: M2023350, a clonal strain of the *Enterococcus avium* with the microbial deposit number of CCTCC NO: M2023350, and a pure culture of the *Enterococcus avium* with the microbial deposit number of CCTCC NO: M2023350; the third probiotic is selected from *Lactobacillus salivarius* with a microbial deposit number of CCTCC NO: M2023348, a progeny strain of the *Lactobacillus salivarius* with the microbial deposit number of CCTCC NO: M2023348, a clonal strain of the *Lactobacillus salivarius* with the microbial deposit number of CCTCC NO: M2023348, and a pure culture of the *Lactobacillus salivarius* with the microbial deposit number of CCTCC NO: M2023348; the fourth probiotic is selected from *Limosilactobacillus fermentum* with a microbial deposit number of CCTCC NO: M2023352, a progeny strain of the *Limosilactobacillus fermentum* with the microbial deposit number of CCTCC NO: M2023352, a clonal strain of the *Limosilactobacillus fermentum* with the microbial deposit number of CCTCC NO: M2023352, and a pure culture of the *Limosilactobacillus fermentum* with the microbial deposit number of CCTCC NO: M2023352; and the fifth probiotic is selected from *Parabacteroides distasonis* with a microbial deposit number of CCTCC NO: M20222033, a progeny strain of the *Parabacteroides distasonis* with the microbial deposit number of CCTCC NO: M20222033, a clonal strain of the *Parabacteroides distasonis* with the microbial deposit number of CCTCC NO: M20222033, and a pure culture of the *Parabacteroides distasonis* with the microbial deposit number of CCTCC NO: M20222033.

The second aspect of the present disclosure provides a microecological composition, which comprises the probiotic composition according to the first aspect of the present disclosure as an active material.

In some embodiments, the composition further comprises an auxiliary material, wherein the auxiliary material is selected from a lyoprotectant, a bacterial culture medium, a food additive, an acceptable carrier or auxiliary material in a healthcare product, and a pharmaceutically acceptable carrier or auxiliary material.

In some embodiments, in the microecological composition, based on the viable count of bacterial cells, the content ratio of any two strains is 100 CFU:(1-10000) CFU (e.g., 100 CFU:any value of 1 CFU, 2 CFU, 3 CFU, 4 CFU, 5 CFU, 6 CFU, 7 CFU, 8 CFU, 9 CFU, 10 CFU, 20 CFU, 30 CFU, 40 CFU, 50 CFU, 60 CFU, 70 CFU, 80 CFU, 90 CFU, 100 CFU, 200 CFU, 300 CFU, 400 CFU, 500 CFU, 600 CFU, 700 CFU, 800 CFU, 900 CFU, 1000 CFU, 2000 CFU, 3000 CFU, 4000 CFU, 5000 CFU, 6000 CFU, 7000 CFU, 8000 CFU, 9000 CFU, and 10000 CFU).

The third aspect of the present disclosure provids a use of the probiotic composition according to the first aspect of the present disclosure or the microecological composition according to the second aspect of the present disclosure in the preparation of a product for use alone or in combination with an additional microbial formulation and/or medicament to improve the health status of a subject, wherein the improvement of the health status of the subject is selected from: inhibiting proliferation of any one, any two, any three, any four, any five, any six, any seven, or eight of *Pseudomonas aeruginosa, Shigella dysenteriae, Staphylococcus aureus, Escherichia coli, Salmonella paratyphi B, Yersinia enterocolitica, Vibrio parahaemolyticus,* and *Clostridioides difficile* in a body cavity (e.g., the intestinal cavity) of the subject; treating, preventing, and/or mitigating tissue damage, diseases, or sub-health status caused by any one, any two, any three, any four, any five, any six, any seven, or eight of *Pseudomonas aeruginosa, Shigella dysenteriae, Staphylococcus aureus, Escherichia coli, Salmonella paratyphi B, Yersinia enterocolitica, Vibrio parahaemolyticus,* and *Clostridioides difficile*; improving the antioxidant capacity in the intestinal tract of the subject; treating, preventing, and/or mitigating diarrhea caused by an anti-tumor drug; treating, preventing, and/or mitigating intestinal inflammation caused by an anti-tumor drug; treating, preventing, and/or mitigating weight loss caused by an anti-tumor drug; treating, preventing, and/or mitigating shortening of small intestine length caused by an anti-tumor drug; treating, preventing, and/or mitigating an increase in small intestine thickness caused by an anti-tumor drug; treating, preventing, and/or mitigating intestinal injury caused by an anti-tumor drug; treating, preventing, and/or mitigating a decrease in spleen-to-body weight ratio caused by an anti-tumor drug; treating, preventing, and/or mitigating tissue damage, diseases, or sub-health status caused by increased expression levels of any one, any two, any three, or four of TNF-α, IL-1β, IL-22, and Bax; treating, preventing, and/or mitigating tissue damage, diseases, or sub-health status caused by decreased expression levels of any one, any two, or three of ZO-1, Occludin, and AQP8; treating, preventing, and/or mitigating diarrhea caused by radiotherapy; treating, preventing, and/or mitigating weight loss caused by radiotherapy; treating, preventing, and/or mitigating intestinal injury caused by radiotherapy; treating, preventing, and/or mitigating intestinal inflammation caused by radiotherapy; and treating, preventing, and/or mitigating a decrease in hematocrit, a decrease in white blood cell count, a decrease in lymphocyte count, and/or a decrease in platelet count caused by an anti-tumor drug.

In some embodiments, the product is a food, a healthcare product, or a pharmaceutical product.

In some embodiments, the subject is selected from a human and a mouse.

In some embodiments, the present disclosure provides use of the probiotic composition according to the first aspect of the present disclosure or the microecological composition according to the second aspect of the present disclosure in the preparation of a medicament for treating and/or preventing a cancer treatment-related toxic and side effect.

In some embodiments, the cancer treatment-related toxic and side effect is diarrhea.

In some embodiments, the cancer treatment-related toxic and side effect is diarrhea caused by an anti-tumor drug, or diarrhea caused by radiotherapy, or intestinal injury caused by radiotherapy.

In some embodiments, the anti-tumor drug is selected from a chemotherapeutic drug, a targeted drug, and an immune checkpoint inhibitor.

In some embodiments, the anti-tumor drug is selected from epirubicin, actinomycin D, doxorubicin, daunorubicin, paclitaxel, docetaxel, albumin-bound paclitaxel, cisplatin, carboplatin, nedaplatin, oxaliplatin, lobaplatin, cyclophosphamide, nitrogen mustard, carmustine, camptothecin, hydroxycamptothecin, topotecan, irinotecan, capecitabine, gemcitabine, methotrexate, 5-fluorouracil, pemetrexed, cytarabine, apatinib, axitinib, cabozantinib, sorafenib, sunitinib, nivolumab, pembrolizumab, and ipilimumab.

The fourth aspect of the present disclosure provids a method for preventing, treating, or mitigating a intestinal disease, which comprises administering to a subject a therapeutically effective amount of the microecological composition according to the second aspect of the present disclosure, wherein the intestinal disease is selected from: intestinal diseases caused by any one, any two, any three, any four, any five, any six, any seven, or eight of *Pseudomonas aeruginosa, Shigella dysenteriae, Staphylococcus aureus, Escherichia coli, Salmonella paratyphi B, Yersinia enterocolitica, Vibrio parahaemolyticus,* and *Clostridioides difficile*; oxidative damage in the intestinal tract; diarrhea caused by an anti-tumor drug; intestinal inflammation caused by an anti-tumor drug; intestinal injury caused by an anti-tumor drug; diarrhea caused by radiotherapy; intestinal inflammation caused by radiotherapy; and intestinal injury caused by radiotherapy.

In some embodiments, the subject is selected from a human and a mouse.

In some embodiments, the administration to the subject is selected from oral administration, intraperitoneal injection, and intragastric administration.

In some embodiments, based on the total bacterial content in the probiotic composition, the therapeutically effective amount is 10⁶⁻¹² CFU (e.g., any value or range between any two values of 1 × 10⁶ CFU, 2 × 10⁶ CFU, 3 × 10⁶ CFU, 4 × 10⁶ CFU, 5 × 10⁶ CFU, 6 × 10⁶ CFU, 7 × 10⁶ CFU, 8 × 10⁶ CFU, 9 × 10⁶ CFU, 1 × 10⁷ CFU, 2 × 10⁷ CFU, 3 × 10⁷ CFU, 4 × 10⁷ CFU, 5 × 10⁷ CFU, 6 × 10⁷ CFU, 7 × 10⁷ CFU, 8 × 10⁷ CFU, 9 × 10⁷ CFU, 1 × 10⁸ CFU, 2 × 10⁸ CFU, 3 × 10⁸ CFU, 4 × 10⁸ CFU, 5 × 10⁸ CFU, 6 × 10⁸ CFU, 7 × 10⁸ CFU, 8 × 10⁸ CFU, 9 × 10⁸ CFU, 1 × 10⁹ CFU, 2 × 10⁹ CFU, 3 × 10⁹ CFU, 4 × 10⁹ CFU, 5 × 10⁹ CFU, 6 × 10⁹ CFU, 7 × 10⁹ CFU, 8 × 10⁹ CFU, 9 × 10⁹ CFU, 1 × 10¹⁰ CFU, 2 × 10¹⁰ CFU, 3 × 10¹⁰ CFU, 4 × 10¹⁰ CFU, 5 × 10¹⁰ CFU, 6 × 10¹⁰ CFU, 7 × 10¹⁰ CFU, 8 × 10¹⁰ CFU, 9 × 10¹⁰ CFU, 1 × 10¹¹ CFU, 2 × 10¹¹ CFU, 3 × 10¹¹ CFU, 4 × 10¹¹ CFU, 5 × 10¹¹ CFU, 6 × 10¹¹ CFU, 7 × 10¹¹ CFU, 8 × 10¹¹ CFU, 9 × 10¹¹ CFU, and 10¹² CFU) per day.

In some embodiments, the anti-tumor drug is selected from epirubicin, actinomycin D, doxorubicin, daunorubicin, paclitaxel, docetaxel, albumin-bound paclitaxel, cisplatin, carboplatin, nedaplatin, oxaliplatin, lobaplatin, cyclophosphamide, nitrogen mustard, carmustine, camptothecin, hydroxycamptothecin, topotecan, irinotecan, capecitabine, gemcitabine, methotrexate, 5-fluorouracil, pemetrexed, cytarabine, apatinib, axitinib, cabozantinib, sorafenib, sunitinib, nivolumab, pembrolizumab, and ipilimumab.

The present disclosure has the following beneficial effects:
(1) The probiotic composition provided by the present disclosure contains 3, 4, or 5 probiotics of different genera/species, and the strains do not exhibit mutual antagonism, are free of virulence factors, and demonstrate good safety; (2) the probiotic composition provided by the present disclosure possesses capabilities of auto-aggregation/co-aggregation, antioxidation, short-chain fatty acid production, and inhibition of multiple pathogenic bacteria, has strong *in vitro* adhesion ability, and can synergistically function against the complex pathogenesis of cancer treatment-related diarrhea; and (3) the probiotic composition provided by the present disclosure can prevent and/or treat toxic and side effects such as diarrhea, intestinal inflammation, and intestinal injury caused by chemotherapeutic drugs or radiotherapy.

### BRIEF DESCRIPTION OF THE DRAWINGS

FIG. 1 shows top-view photographs illustrating the colony morphology of 5 strains.
FIG. 2 shows photographs illustrating co-culture characteristics of five single strains on BF839 agar media.
FIG. 3 shows the auto-aggregation (co-aggregation) ability of the probiotic composition and single strains thereof.
FIG. 4 shows the detection results of the antibacterial activity of the probiotic composition and single strains thereof against pathogenic bacteria.
FIG. 5 shows the detection results of the adhesion ability of the probiotic composition and single strains thereof to Caco2 cells.

### DETAILED DESCRIPTION

In order to make the purposes, technical solutions, and advantages of the present disclosure clearer, the embodiments of the present disclosure will be further described in detail with reference to the accompanying drawings.

Unless otherwise specified, the terms used herein have the meanings commonly understood in the biomedical field.

Unless otherwise specified, the specific temperature parameters in the present disclosure should be understood as constant-temperature treatment, and it is permissible for variations to occur within a certain temperature range (e.g., fluctuations within the range of ±5 °C, ±4 °C, ±3 °C, ±2 °C, or ±1 °C).

The present disclosure provides a probiotic composition, which comprises any three, any four, or five of *Bifidobacterium bifidum, Enterococcus avium, Lactobacillus salivarius (Ligilactobacillus salivarius), Limosilactobacillus fermentum,* and *Parabacteroides distasonis.*

In some embodiments, the *Bifidobacterium bifidum* has a microbial deposit number of CCTCC NO: M2023349;
the *Enterococcus avium* has a microbial deposit number of CCTCC NO: M2023350;
the *Lactobacillus salivarius* has a microbial deposit number of CCTCC NO: M2023348;
the *Limosilactobacillus fermentum* has a microbial deposit number of CCTCC NO: M2023352; and
the *Parabacteroides distasonis* has a microbial deposit number of CCTCC NO: M20222033.

The meanings of the strains with the specific microbial deposit numbers of the present disclosure include, but are not limited to: (1) strains with the specific microbial deposit numbers deposited in the depository center; (2) strains having the same genome as the strains in (1); (3) passaged strains based on the aforementioned (1) or (2) that have no gene mutations; (4) passaged strains based on the aforementioned (1), (2), or (3) that have accumulated minor mutations during passaging, but whose toxicity, immunogenicity, and bioactivity exhibit no substantial changes; and (5) live bacteria based on any one of the strains in the aforementioned (1) to (4), inactivated substances of the live bacteria, lysates of the live bacteria, fermentation products of the live bacteria, or the like.

The strains having the same genome include, but are not limited to, strains having the same genetic background independently isolated and disclosed by others after the corresponding priority date of the present disclosure, i.e., strains isolated from nature or animals (including humans) having the same genome (the same genetic background). Conventional cultures are generally considered to be passaged strains without gene mutations. As is known to those skilled in the art, minor mutations are generally inevitably introduced when strains are passaged. When mutations occur in non-coding sequence regions, or are synonymous mutations in coding regions, or are mutations that do not affect the toxicity (biosafety), immunogenicity, and bioactivity of the strains (for example, it is possible that the mutations occur at linker amino acid residues between two domains or that the mutated amino acid residues are located inside the protein's higher-order structure; due to the absence of contact with immune cells, these mutations do not affect the toxicity, immunogenicity, and bioactivity), it can be reasonably expected that in the case that these minor changes do not significantly affect the toxicity, immunogenicity, and bioactivity of the progeny strains, the mutated strains can still achieve the objective of the present disclosure, and the mutated strains are derived from the strains contributed by the present disclosure, so the corresponding strains still fall within the substantial technical contribution scope of the present disclosure. These minor mutations are still insubstantial mutations, and the strains with these minor mutations should be considered as mutated strains that have no changes in toxicity, immunogenicity, and bioactivity. From the detection perspective, the absence of substantial changes in toxicity, immunogenicity, and bioactivity of strains includes, but is not limited to, circumstances where, within the scope of limitations of detection techniques (such as detection sensitivity and detection limits) and the range of acceptable or inevitable errors, the toxicity, immunogenicity, and bioactivity of the mutated strains are deemed the same as those of the strains contributed by the present disclosure. When the toxicity, immunogenicity, and bioactivity of the progeny of the strains are determined using cells, animals, etc., differences reflected in cell lines, animal species, age, sex, health status, culture conditions, etc., as well as expected or inevitable systematic errors, fall under the scope of having no substantial changes. The active ingredients refer to substances that function as components to produce biological effects. In the present disclosure, the active ingredients are the probiotic strains. Through the research on the co-culture characteristics of the five strains in the present disclosure, it can be found that no mutual inhibition exists between any two of the strains; therefore, compositions containing any 3, any 4, or 5 of the strains can be formulated according to the efficacy characteristics of these strains, and it is reasonably expected that the compositions formulated with these strains can simultaneously exert the efficacy of the strains in the combination.

Cancer treatment-related diarrhea or tumor-associated diarrhea refers to diarrhea caused by damage to the intestinal mucosa from various anti-tumor treatments that leads to an imbalance in intestinal absorption and secretion. Common cancer treatment-related diarrhea includes chemotherapy-induced diarrhea, diarrhea caused by radiotherapy, diarrhea caused by targeted therapy, diarrhea caused by an immune checkpoint inhibitor, and the like. Diarrhea caused by radiotherapy clinically manifests as symptoms of radiation enteritis or radiation intestinal injury.

Chemotherapy refers to the use of non-selective chemical drug means to kill tumor cells to achieve therapeutic purposes. Chemotherapy is a main approach for tumor treatment besides surgery and radiotherapy. Due to the lack of selectivity, chemotherapy may also damage normal cells while killing tumor cells. Common chemotherapeutic drugs include, but are not limited to, antibiotic-based chemotherapeutic drugs (e.g., epirubicin, actinomycin D, doxorubicin, daunorubicin, and derivatives thereof, etc.), paclitaxel-based chemotherapeutic drugs (e.g., paclitaxel, docetaxel, albumin-bound paclitaxel, and derivatives thereof, etc.), platinum-based chemotherapeutic drugs (e.g., cisplatin, carboplatin, nedaplatin, oxaliplatin, lobaplatin, and derivatives thereof, etc.), alkylating agent-based chemotherapeutic drugs (e.g., cyclophosphamide, nitrogen mustard, carmustine, and derivatives thereof, etc.), camptothecin-based chemotherapeutic drugs (e.g., camptothecin, hydroxycamptothecin, topotecan, irinotecan, and derivatives thereof, etc.), and antimetabolite chemotherapeutic drugs (e.g., capecitabine, gemcitabine, methotrexate, 5-fluorouracil, pemetrexed, cytarabine, and derivatives thereof, etc.).

Targeted therapy refers to designing corresponding therapeutic drugs against already defined oncogenic sites at the cellular molecular level. In targeted therapy, drugs entering the body specifically select and bind to oncogenic sites to take effect, thereby causing specific death of tumor cells. The emergence of novel targeted drugs has transformed tumor treatment modalities and inaugurated the era of targeted therapy. Common targeted drugs include apatinib, axitinib, cabozantinib, sorafenib, sunitinib, etc.

Immune checkpoint inhibitors are some monoclonal antibody drugs developed against corresponding immune checkpoints. The main role of immune checkpoint inhibitors is to block the interaction between tumor cells expressing immune checkpoints and immune cells, thereby blocking the inhibitory effect of tumor cells on immune cells. Common immune checkpoint inhibitors include, but are not limited to, PD-1/PD-L1 inhibitor antibodies (e.g., nivolumab, pembrolizumab, etc.) and CTLA-4 inhibitors (e.g., ipilimumab, etc.).

Radiotherapy refers to a method for treating malignant tumors using radiation (e.g., α-rays, β-rays, and γ-rays generated by radioisotopes, and X-rays, electron beams, proton beams, and other particle beams generated by various X-ray treatment devices or accelerators, etc.). Common radiotherapy methods include, but are not limited to, conventional radiotherapy, stereotactic radiotherapy, three-dimensional conformal radiotherapy, intensity-modulated radiotherapy, image-guided radiotherapy, volumetric modulated arc radiotherapy, proton radiotherapy, etc.

The present disclosure provides a microecological composition, which comprises: any three, any four, or five of *Bifidobacterium bifidum, Enterococcus avium, Lactobacillus salivarius (Ligilactobacillus salivarius), Limosilactobacillus fermentum,* and *Parabacteroides distasonis*; and an auxiliary material.

The microecological composition refers to a biologically active formulation, which comprises specific microbial species and/or metabolites thereof. The composition restores or optimizes the structure and function of the microbial community in the host, thereby promoting the host's health status, enhancing the resistance to diseases, or improving the host's physiological functions.

The auxiliary materials vary depending on the type of product being prepared, for example, a food, a healthcare product, or a pharmaceutical product may be prepared; correspondingly, the auxiliary material may be selected from a lyoprotectant, a bacterial culture medium, a food additive, an acceptable carrier or auxiliary material in a healthcare product, and a pharmaceutically acceptable carrier or auxiliary material.

The present disclosure provides use of the probiotic composition or the microecological composition in the preparation of a food, a healthcare product, or a pharmaceutical product.

When prepared as a pharmaceutical product, the probiotic composition or the microecological composition is used in the preparation of a pharmaceutical product for use alone or in combination with an additional microbial formulation and/or medicament to improve the health status of a subject.

The present disclosure further provides a method for preventing, treating, or mitigating an intestinal disease, which comprises administering to a subject a therapeutically effective amount of the microecological composition.

The therapeutically effective amount or prophylactically effective amount is an amount that can clinically achieve the desired therapeutic or prophylactic effect. In some embodiments, the therapeutically effective amount does not induce or cause undesired side effects. In some embodiments, the therapeutically effective amount induces or causes side effects, but only those side effects that are considered clinically acceptable by the clinician in view of the patient's condition and treatment. In some embodiments, a single administration dose or a single effective amount contains a total bacterial count of 10²-10¹⁵ CFU, 10³-10¹⁴ CFU, 10⁴-10¹³ CFU, 10⁵-10¹² CFU, or 10⁶-10¹² CFU.

The pharmaceutically acceptable carrier refers to a pharmaceutical carrier that does not cause significant irritation to a subject and does not eliminate the bioactivity and characteristics of the administered probiotics. As is known to those skilled in the art (see, e.g., Remington's Pharmaceutical Sciences, 18th Ed., Mack Printing Company, 1990, pp. 1289-1329), a pharmaceutically acceptable carrier can enhance or stabilize a composition, or can be used to facilitate preparation of a composition. The pharmaceutically acceptable carrier may include a solvent, a dispersion medium, a coating, a surfactant, an antioxidant, an isotonic agent, an absorption delaying agent, a salt, a pharmaceutical stabilizer, a binding agent, an excipient, a disintegrant, a lubricant, a sweetening agent, a flavoring agent, a dye, a protective agent, etc., and a combination thereof. Except insofar as a conventional carrier is incompatible with the active ingredients, its use in the therapeutic or pharmaceutical compositions is contemplated. The carrier can be selected to minimize adverse side effects on the subject and/or to minimize inactivation of the active ingredients.

The excipient refers to a substance added to a pharmaceutical composition to give the drug a certain shape or a certain concentration, for example, sterile water, normal saline, polyalkylene glycol (e.g., polyethylene glycol), vegetable oil, hydrogenated naphthalene, calcium bicarbonate, calcium phosphate, various sugars, various types of starch, cellulose derivatives, gelatin, and the like.

The microecological composition of the present disclosure may further comprise a second beneficial active ingredient, for example, an additional probiotic, prebiotic, drug, etc., having antidiarrheal function. Prebiotics help regulate the intestinal environment by promoting the growth of probiotics in the intestinal tract, thereby indirectly exerting an antidiarrheal effect. Examples of the second beneficial active ingredient include, but are not limited to, *Bacillus licheniformis, Bifidobacterium, Clostridium butyricum,* fructo-oligosaccharide, galacto-oligosaccharide, isomalto-oligosaccharide, xylo-oligosaccharide, mannan oligosaccharide, inulin, stachyose, soybean oligosaccharide, β-glucan, lactosucrose, and the like.

In some embodiments, the pharmaceutical composition is in a dosage form of a tablet, a capsule, a granule, a solution, a suspension, a powder, or the like.

The technical solutions in the examples of the present disclosure will be described clearly and completely below. It is apparent that the examples described herein are only some, but not all, examples of the present disclosure. All other examples obtained by those skilled in the art based on the examples in the application documents of the present disclosure without creative efforts shall fall within the disclosure or protection scope of the present disclosure.

The media used in the following examples were prepared as follows:
Preparation of YCFA liquid medium: 10.0 g of peptone, 2.5 g of a yeast extract, 0.45 mL of a 10% (w/w) aqueous MgSO₄·7H₂O solution, 0.45 mL of a 10 mg/mL aqueous CaCl₂ solution, 10 mL of TE141, 0.45 g of K₂HPO₄, 0.45 g of KH₂PO₄, 0.90 g of NaCl, and 3.2 mL of VFA-mix were added to 1 L of distilled water to obtain a solution. The solution was purged with N₂ to remove oxygen and aliquoted. The aliquoted solution was subjected to moist heat sterilization at a high temperature of 121 °C for 30 min for later use.

Preparation of TE141: 1.50 g of nitrilotriacetic acid was added to 200 mL of purified water to obtain a solution. An appropriate amount of NaOH was added to the solution until the solution became clear, followed by the addition of 800 mL of water, and then the pH value was adjusted to 5.5 with 50% HCl, thus obtaining an aqueous nitrilotriacetic acid solution. 3.00 g of MgSO₄·7H₂O, 0.50 g of MnSO₄·H₂O, 1.00 g of NaCl, 0.10 g of FeSO₄·7H₂O, 0.18 g of CoSO₄·7H₂O, 0.10 g of CaCl₂·2H₂O, 0.18 g of ZnSO₄·7H₂O, 0.006 g of CuSO₄·5H₂O, 0.02 g of KAl(SO₄)₂·12H₂O, 0.01 g of H₃BO₃, 0.01 g of Na₂MoO₄·2H₂O, 0.03 g of NiCl₂·6H₂O, 0.03 mL of a 10 mg/mL Na₂SeO₃·5H₂O solution, and 0.03 mL of a 10 mg/mL Na₂WO₄·2H₂O solution were added to the above aqueous nitrilotriacetic acid solution (during the addition, the solution was continuously stirred to keep clear) to obtain TE141.

Preparation of VFA-mix: 90 mL of acetic acid, 30 mL of propionic acid, 10 mL of n-valeric acid, 10 mL of isobutyric acid, and 10 mL of butyric acid were mixed well to obtain a solution for later use. The aforementioned solution was adjusted to neutrality with 5 M NaOH before use.

Preparation of three-component mixed liquid medium (BHI + MRS + modified GAM): 19.25 g of BHI broth powder (Qingdao Hi-Tech Industrial Park Hope Bio-Technology Co., Ltd., HB8297-5), 13.5 g of MRS broth powder (Guangdong Huankai Biotechnology Co., Ltd., 027312), and 15 g of modified GAM broth powder (Qingdao Hi-Tech Industrial Park Hope Bio-Technology Co., Ltd., HB8518-3) were dissolved in 1 L of distilled water to obtain a solution. The solution was purged with N₂ to remove oxygen and aliquoted. The aliquoted solution was subjected to moist heat sterilization at a high temperature of 121 °C for 30 min. The resulting three-component mixed liquid medium was stored in a shady, dry place.

Preparation of three-component mixed solid medium: 5 g of agar powder was added on the basis of the aforementioned three-component mixed liquid medium, and the other steps were the same as those in the preparation of the three-component mixed liquid medium.

Preparation of BF839 liquid medium: 6.0 g of potato extract powder (Beijing Solarbio Science & Technology Co., Ltd., FA0270), 10.0 g of multivalent peptone (Beijing Solarbio Science & Technology Co., Ltd., P8950-250), 5.0 g of proteose peptone (Qingdao Hi-Tech Industrial Park Hope Bio-Technology Co., Ltd., HB8277), 0.3 g of sodium thioglycolate (Shanghai Aladdin Biochemical Technology Co., Ltd., S105664-25G), 5.0 g of yeast extract powder (Thermo Fisher Oxoid, LP0021B), 1.5 g of glucose (Chengdu Kelong Chemical Co., Ltd., 50-99-7), and 4.0 g of disodium hydrogen phosphate (Chengdu Kelong Chemical Co., Ltd., 7558-79-4) were dissolved in 1 L of distilled water to obtain a solution. The solution was purged with N₂ to remove oxygen and aliquoted. The solution was subjected to moist heat sterilization at a high temperature of 121 °C for 15 min. The resulting medium was stored in a shady, dry place.

Preparation of BF839 solid medium: 50.4 g of BF839 solid agar (Qingdao Hi-Tech Industrial Park Hope Bio-Technology Co., Ltd., HB8805) was dissolved in 1 L of distilled water to obtain a mixture. The mixture was purged with N₂ to remove oxygen and aliquoted. The mixture was subjected to moist heat sterilization at a high temperature of 121 °C for 15 min. The resulting medium was stored in a shady, dry place.

Preparation of oxygen-free PBS: 0.27 g of potassium dihydrogen phosphate, 1.42 g of disodium hydrogen phosphate, 8 g of sodium chloride, and 0.2 g of potassium chloride were dissolved in 1 L of distilled water to obtain a mixture. The mixture was heated to boiling and then cooled to room temperature, and 0.55 g of cysteine hydrochloride was added thereto. The resulting mixture was stirred for dissolution, and then the pH was adjusted to 6.5. The mixture was heated to boiling and kept at a slight boiling state for 30 min. The mixture was cooled and then aliquoted into bottles at 400 mL/bottle using a quantitative dispenser under N₂ atmosphere. The bottles were subjected to moist heat sterilization at a high temperature of 121 °C for 30 min. The resulting PBS was stored in a shady, dry place for later use.

The preparation of GAM solid medium (Qingdao Hi-Tech Industrial Park Hope Bio-Technology Co., Ltd., HB8462), TSB medium (tryptone soy broth, Qingdao Hi-Tech Industrial Park Hope Bio-Technology Co., Ltd., HB4114), and TSA medium (tryptone soy agar, Qingdao Hi-Tech Industrial Park Hope Bio-Technology Co., Ltd., HB4138) was carried out according to the procedures specified in the instructions provided by the supplier, where the materials were weighed and dissolved in water to obtain a mixture. Each mixture was subjected to moist heat sterilization at a high temperature of 121 °C for 30 min to obtain a medium. The medium was stored in a shady, dry place.

Preparation of bacterial powder preparation medium for *Parabacteroides distasonis* Pdist-1: 6 g of anhydrous glucose, 15 g of soy peptone, 10 g of yeast extract powder, 10 g of yeast peptone, 2 g of potassium dihydrogen phosphate, 2 g of disodium hydrogen phosphate, 0.2 g of magnesium sulfate, 0.01 g of manganese sulfate, 0.2 g of calcium chloride, 1 mL of Tween 80, and 0.5 g of cysteine hydrochloride monohydrate were dissolved in 1 L of distilled water. The mixed solution was purged with N₂ to remove oxygen and aliquoted. The mixed solution was sterilized at 121 °C for 15 min.

Preparation of bacterial powder preparation medium for *Limosilactobacillus fermentum* Lferm-1: 30 g of anhydrous glucose, 15 g of soy peptone, 10 g of yeast extract powder, 5 g of sodium acetate, 2 g of potassium dihydrogen phosphate, 2 g of disodium hydrogen phosphate, 0.1 g of magnesium sulfate, 0.045 g of manganese sulfate, 1 mL of Tween 80, and 0.5 g of cysteine hydrochloride monohydrate were dissolved in 1 L of purified water. The mixed solution was purged with N₂ to remove oxygen and aliquoted. The mixed solution was subjected to moist heat sterilization at a high temperature of 121 °C for 15 min.

Preparation of bacterial powder preparation medium for *Lactobacillus salivarius* Lsali-1: 24 g of anhydrous glucose, 20 g of soy peptone, 10 g of yeast extract powder, 10 g of peptone, 5 g of sodium acetate, 2 g of potassium dihydrogen phosphate, 2 g of disodium hydrogen phosphate, 0.1 g of magnesium sulfate, 0.045 g of manganese sulfate, 1 mL of Tween 80, and 0.5 g of cysteine hydrochloride monohydrate were dissolved in 1 L of purified water. The mixed solution was purged with N₂ to remove oxygen and aliquoted. The mixed solution was subjected to moist heat sterilization at a high temperature of 121 °C for 15 min.

Preparation of bacterial powder preparation medium for *Enterococcus avium* Eaviu-1: 30 g of anhydrous glucose, 15 g of soy peptone, 10 g of yeast powder, 5 g of sodium acetate, 2 g of dipotassium hydrogen phosphate, 0.1 g of magnesium sulfate, 0.045 g of manganese sulfate, 1 mL of Tween 80, and 0.5 g of cysteine hydrochloride monohydrate were dissolved in 1 L of purified water. The mixed solution was purged with N₂ to remove oxygen and aliquoted. The mixed solution was subjected to moist heat sterilization at a high temperature of 121 °C for 15 min.

Preparation of bacterial powder preparation medium for *Bifidobacterium bifidum* Bbifi-1: 20 g of anhydrous glucose, 40 g of soy peptone, 5 g of *N-*acetylglucosamine, 2 g of potassium dihydrogen phosphate, 2 g of disodium hydrogen phosphate, 0.1 g of magnesium sulfate, 0.045 g of manganese sulfate, 1 mL of Tween 80, and 0.5 g of cysteine hydrochloride monohydrate were dissolved in 1 L of purified water. The mixed solution was purged with N₂ to remove oxygen and aliquoted. The mixed solution was subjected to moist heat sterilization at a high temperature of 121 °C for 15 min.

The lyoprotectants were prepared as follows:
Preparation of lyoprotectant for *Parabacteroides distasonis* Pdist-1 (which also served as a lyoprotectant control for animal administration): Solution A: 6 g of sucrose, 6 g of trehalose, 2 g of xylitol, 2 g of sorbitol, and 44 g of purified water, sterilized at 115 °C for 30 min. Solution B: 5 g of sodium glutamate and 15 g of purified water; sterilized at 115 °C for 20 min. Solution C: 4 g of sodium ascorbate and 16 g of purified water. These solutions were filtered and sterilized for later use.

Preparation of lyoprotectants for *Limosilactobacillus fermentum* Lferm-1, *Lactobacillus salivarius* Lsali-1, and *Enterococcus avium* Eaviu-1: Solution A: 8 g of sucrose, 8 g of trehalose, and 44 g of purified water, sterilized at 115 °C for 30 min. Solution B: 2 g of sodium glutamate, 2 g of arginine hydrochloride, and 16 g of purified water, sterilized at 115 °C for 30 min. Solution C: 4 g of sodium ascorbate and 16 g of purified water. These solutions were filtered and sterilized for later use.

Preparation of lyoprotectant for *Bifidobacterium bifidum* Bbifi-1: Solution A: 6 g of sucrose, 6 g of trehalose, 2 g of xylitol, 2 g of sorbitol, and 44 g of purified water, sterilized at 115 °C for 30 min. Solution B: 2 g of arginine hydrochloride, 2 g of sodium glutamate, and 16 g of purified water, sterilized at 115 °C for 30 min. Solution C: 4 g of sodium ascorbate and 16 g of purified water. These solutions were filtered and sterilized for later use.

When in use, solution A, solution B, and solution C were mixed in a volume ratio of 6:2:2. The prepared lyoprotectant solution was lyophilized, then pulverized, and formulated into a suspension with normal saline to serve as the lyoprotectant for administration in an animal experiment.

Preparation of 0.1% Tween 80-PBS dilution: 3.58 g of disodium hydrogen phosphate dodecahydrate, 0.27 g of potassium dihydrogen phosphate, 8 g of sodium chloride, and 1 mL of Tween 80 were added to 1 L of boiling water and dissolved by stirring with a glass rod. 0.5 g of cysteine hydrochloride monohydrate was added to the aforementioned boiled solution. A Hungate apparatus was opened, and the solution was boiled again under N₂ atmosphere. After being purged with N₂ for 20 min, the solution was aliquoted into anaerobic bottles purged with N₂ for oxygen removal. The bottles were capped with stoppers and labeled, and the solutions were sterilized at a high temperature of 121 °C for 15 min.

### Example 1: Strain Isolation and Identification

Fresh stool samples were collected from several healthy human volunteers, and each stool sample was independently processed. An appropriate amount of oxygen-free PBS was added to the stool sample to obtain a mixture, and the mixture was shaken to obtain a suspension. The suspension was filtered with gauze under N₂ atmosphere to obtain a filtrate. The filtrate was centrifuged at 10000 rpm for 20 min, then the supernatant was discarded, and the pellet was retained. An appropriate amount of oxygen-free PBS was added to the pellet to resuspend the bacterial cells, thus obtaining a suspension. An equal volume of a 50% (v/v) aqueous oxygen-free glycerol solution was added to the suspension, and the mixture was mixed well to obtain a bacterial mixed solution sample. The sample was aliquoted into sample tubes, and the sample tubes were sealed in bags and vacuumized, and then stored in a freezer at -80 °C. The sample in each cryopreserved sample tube was thawed independently. 0.5 mL of the thawed sample was resuspended in 4.5 mL of oxygen-free PBS, and the mixture was mixed well by shaking to obtain a bacterial suspension. Under anaerobic conditions, 0.5 mL of the bacterial suspension and 4.5 mL of anaerobic PBS were mixed well for dilution by shaking. The resulting solution was serially diluted ten-fold to a 10⁻⁶ dilution ratio using the same method. The bacterial solution with an appropriate dilution ratio was taken and well mixed with a YCFA liquid medium, the resulting mixture was then aliquoted into wells of a 384-well plate, and the plate was anaerobically incubated at 37 °C for one week. The bacterial solution in a well where the bacteria had grown was inoculated into the YCFA medium and cultured for 48 h, and then the bacterial solution was aliquoted into two aliquots. One aliquot of the bacterial solution was examined by MALDI-TOF-MS for preliminary species classification of the isolated strain. After it was determined that the bacterial solution contained only bacteria with one genetic background (monoclonal strain), another aliquot of the bacterial solution was further inoculated into the YCFA medium for culture according to the mass spectrometry results, where the 16S rDNA gene amplification and sequencing were performed on one aliquot; and a 50% (v/v) aqueous glycerol solution was added to another aliquot in a volume ratio of 1:1, and the mixture was well mixed and deposited.

The 16S rDNA gene sequence obtained by sequencing was aligned with the NCBI Nucleotide database to further identify the species of the isolated strain. From numerous strains with further confirmed species, 5 strains were selected for subsequent experiments of the present disclosure. Strain 1 had the highest sequence similarity (> 99%) to a strain of *Parabacteroides distasonis.* Therefore, strain 1 was named *Parabacteroides distasonis* Pdist-1 (abbreviated as Pdist-1). Strain 2 had the highest sequence similarity (100%) to a strain of *Limosilactobacillus fermentum.* Therefore, strain 2 was named *Limosilactobacillus fermentum* Lferm-1 (abbreviated as Lferm-1). Strain 3 had the highest sequence similarity (100.00%) to a strain of *Lactobacillus salivarius* (*Lactobacillus salivarius* is also called *Ligilactobacillus salivarius*)*.* Therefore, strain 3 was named *Lactobacillus salivarius* Lsali-1 (abbreviated as Lsali-I). Strain 4 had the highest sequence similarity (100.00%) to a strain of *Enterococcus avium.* Therefore, strain 4 was named *Enterococcus avium* Eaviu-1 (abbreviated as Eaviu-1). Strain 5 had the highest sequence similarity (99.86%) to a strain of *Bifidobacterium bifidum.* Therefore, strain 5 was named *Bifidobacterium bifidum* Bbifi-1 (abbreviated as Bbifi-1).

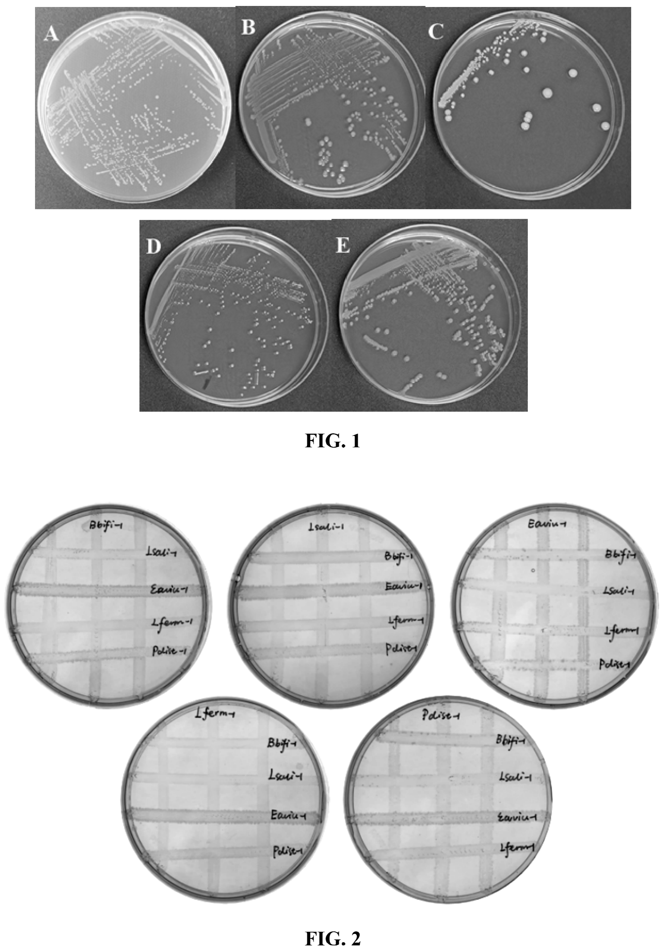

*Parabacteroides distasonis* Pdist-1, *Limosilactobacillus fermentum* Lferm-1, and *Enterococcus avium* Eaviu-1 were each inoculated into a BF839 medium and cultured to observe their colony morphology. *Lactobacillus salivarius* Lsali-1 and *Bifidobacterium bifidum* Bbifi-1 were each inoculated into a three-component mixed solid medium and cultured to observe their colony morphology. The top-view photographs illustrating the colony morphology of the aforementioned 5 strains are shown in FIG. 1, where A is a top-view photograph illustrating the colony morphology of *Parabacteroides distasonis* Pdist-1; B is a top-view photograph illustrating the colony morphology of *Limosilactobacillus fermentum* Lferm-1; C is a top-view photograph illustrating the colony morphology of *Lactobacillus salivarius* Lsali-1; D is a top-view photograph illustrating the colony morphology of *Enterococcus avium* Eaviu-1; and E is a top-view photograph illustrating the colony morphology of *Bifidobacterium bifidum* Bbifi-1. It can be seen that the 5 strains all exhibited white opaque round colonies with convex in the middle and smooth and moist surfaces.

### Example 2: Whole Genome Analysis of Strains

The 5 strains obtained in Example 1 were each inoculated into a three-component mixed liquid medium, and the bacteria were cultured to the late logarithmic growth phase. The whole genome DNA of each strain was extracted and subjected to whole genome sequencing using the Illumina high-throughput sequencing platform NovaSeq 6000. After genome sequence assembly and annotation, the protein sequence was input into the virulence factor databases (VFDB) for virulence factor analysis. The results showed that none of the 5 strains had virulence factors in their genomes.

Novelty analysis of the 5 strains was carried out using the average nucleotide identity (ANI) method. Whole genome searches were performed in Genbank and the most similar strains were compared by fastANI (v1.33). The two strains with the highest whole genome similarity to *Parabacteroides distasonis* Pdist-1 were GCA_003462945.1 (ANI = 98.26%) and GCA_003459965.1 (ANI = 98.20%), respectively. The two strains with the highest whole genome similarity to *Limosilactobacillus fermentum* Lferm-1 were GCA_003465085.1 (ANI = 99.32%) and GCA_024385625.1 (ANI = 99.29%), respectively. The two strains with the highest whole genome similarity to *Lactobacillus salivarius* Lsali-1 were GCA_009863605.1 (ANI = 99.94%) and GCA_009866185.1 (ANI = 99.87%), respectively. The two strains with the highest whole genome similarity to *Enterococcus avium* Eaviu-1 were GCA_018917545.1 (ANI = 98.75%) and GCA_018373135.1 (ANI = 98.62%), respectively. The two strains with the highest whole genome similarity to *Bifidobacterium bifidum* Bbifi-1 were GCA_003466395.1 (ANI = 99.01%) and GCA_003437945.1 (ANI = 99.00%), respectively. It can be seen that the species classification made in Example 1 was correct.

The strains Pdist-1, Eaviu-1, Lferm-1, Bbifi-1, and Lsali-1 isolated and cultured in the present disclosure were each submitted to a depository institution recognized by the patent procedure for deposition. The depository institution was the China Center for Type Culture Collection (CCTCC); the address is Wuhan University, Wuhan, China; the culture names, classification and designation, dates of deposition, dates of viability test, and microbial deposit numbers are shown in Table 1.

**Table 1. Summary of strain deposition information**

| Culture name | Classification and designation | Date of deposition | Date of viability test | Microbial deposit number |
|---|---|---|---|---|
| *Parabacteroides distasonis* Pdist-1 | *Parabacteroides distasonis* | 2022-12-23 | 2022-12-30 | CCTCC NO:M20222033 |
| *Enterococcus avium* Eaviu-1 | *Enterococcus avium* | 2023-03-17 | 2023-03-24 | CCTCC NO:M2023350 |
| *Limosilactobacillus fermentum* Lferm-1 | *Limosilactobacillus fermentum* | 2023-03-17 | 2023-03-24 | CCTCC NO:M2023352 |
| *Bifidobacterium bifidum* Bbifi-1 | *Bifidobacterium bifidum* | 2023-03-17 | 2023-03-24 | CCTCC NO:M2023349 |
| *Lactobacillus salivarius* Lsali-1 | *Lactobacillus salivarius* | 2023-03-17 | 2023-03-24 | CCTCC NO:M2023348 |

### Example 3: Test of Co-Culture Characteristics Among Strains

The strains Pdist-1, Eaviu-1, Lferm-1, Bbifi-1, and Lsali-1 obtained in Example 1 were each activated and cultured to the late logarithmic growth phase. The bacterial solution of one of the strains was streaked three times in parallel on a BF839 solid medium using a disposable sterile cotton swab, and then the bacterial solutions of the other four strains were streaked once in parallel in a direction perpendicular to the first streak. After the streaked bacterial solutions were air-dried, the bacteria were anaerobically cultured for 48 h until traces of the bacterial solutions became obvious. The interaction relationships among the five strains are shown in FIG. 2. It can be seen that there were no breakpoints at the intersections of the strains, indicating that no growth inhibition occurred among the strains.

### Example 4: Aggregation Ability of Single Strains and Bacterial Composition

Preparation of single-strain bacterial suspensions: The strains Pdist-1, Eaviu-1, Lferm-1, Bbifi-1, and Lsali-1, and the control strain *Lactobacillus rhamnosus* GG (LGG, CICC6141, purchased from the China Center of Industrial Culture Collection) were each inoculated into a three-component mixed liquid medium at an initial inoculation amount of 2%, and then anaerobically cultured at 37 °C to the late logarithmic growth phase. The cultured bacterial solutions were centrifugally washed 3 times with oxygen-free PBS. The bacterial cells were resuspended in an appropriate amount of PBS, and then the OD₆₀₀ of the bacterial solutions was determined (the absorbance of the bacterial solutions at a wavelength of 600 nm was determined). The bacterial solutions of the single strains were diluted to the same OD₆₀₀ value (0.5 ± 0.1) using PBS as the diluent.

Preparation of bacterial composition suspension: The bacterial solutions of strains Pdist-1, Eaviu-1, Lferm-1, Bbifi-1, and Lsali-1 that had been diluted to the same OD₆₀₀ value were mixed in equal volumes.

15 mL of each of the diluted single-strain bacterial suspensions and the mixed bacterial suspension was taken and aliquoted into a total of 3 tubes at 5 mL/tube for parallel experiments. The OD₆₀₀ of each bacterial solution at the initial time was measured and recorded as A₀; after the bacterial solution was left to stand at 37 °C for 24 h, 1 mL of the upper-layer bacterial suspension was pipetted, and its OD₆₀₀ value was measured again and recorded as A₂₄. Auto-aggregation rate/co-aggregation rate = (1 - A₂₄/A₀) × 100%. The detection results were expressed as Mean ± S.D. (auto-aggregation rate: the aggregation rate between same strains; co-aggregation rate: the aggregation rate between different strains in the bacterial composition).

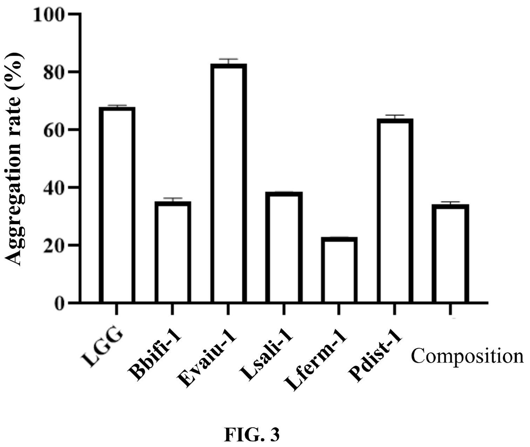

The results are shown in FIG. 3. It can be seen that the bacterial composition and the single strains thereof all had the aggregation ability, and the order of their aggregation ability (auto-aggregation rate/co-aggregation rate, aggregation rate) was as follows: Eaviu-1 > LGG ≈ Pdist-1 > Lsali-1 ≈ Bbifi-1 ≈ bacterial composition > Lferm-1. The bacteria with better aggregation ability may have better colonization ability and readily form a protective barrier on the surface of host intestinal cells.

### Example 5: Test of Antioxidant Capacity of Bacterial Composition

Culture of bacterial composition: The single strains Pdist-1, Eaviu-1, Lferm-1, Bbifi-1, and Lsali-1 were activated and then mixed and inoculated into 5 mL of a three-component mixed medium, with the inoculation amount of each strain being 1%. The mixture was anaerobically cultured at 37 °C for 24 h.

Sample treatment: 0.5 mL of the cultured bacterial solution was taken and centrifuged at 12000 rpm for 20 min, the supernatant was discarded, and the bacterial cells were resuspended in 0.5 mL of pre-cooled extraction solution in a total antioxidant capacity assay kit for strains (the kit was purchased from Beijing Solarbio Science & Technology Co., Ltd., BC1315). The bacterial cells were transferred into a sterilized screw cap tube containing beads (Sigma-Aldrich, USA, G4649-1KG). The bacterial cells were then subjected to one round of cell wall disruption by shaking using a rapid sample preparation instrument (parameter setting: 4.5 m/s, 30 s), followed by centrifugation at 12000 rpm and 4 °C for 10 min. The supernatant was collected and placed on ice for subsequent testing.

A BCA protein concentration assay kit (the kit was purchased from Beijing Solarbio Science & Technology Co., Ltd., PC0020) was used; according to the instructions of the kit, a standard curve was plotted, and the BCA sample was assayed.

The antioxidant capacity of the sample was assayed using the total antioxidant capacity assay kit for strains according to the instructions of the kit in combination with the aforementioned standard curve. The unit of the total antioxidant capacity is µmol/mg prot.

The antioxidant capacity of the bacterial composition was 0.189 µmol/mg prot. The bacterial composition possessed certain antioxidant capacity.

### Example 6: Ability of Single Strains and Bacterial Composition to Produce Short-Chain Fatty Acids

Single strains: The control strain LGG, and the strains Pdist-1, Eaviu-1, Lferm-1, Bbifi-1, and Lsali-1 were each inoculated into a BF839 liquid medium at an inoculation amount of 5%, and then anaerobically cultured at 37 °C for 24 h.

Bacterial composition: Pdist-1, Eaviu-1, Lferm-1, Bbifi-1, and Lsali-1 were mixed and inoculated into a BF839 liquid medium, with the inoculation amount of each strain being 1%. The mixture was anaerobically cultured at 37 °C for 24 h.

Each of the fermentation broths of the single strains and the bacterial composition was centrifuged, and 1 mL of the supernatant was pipetted. 10 µL of formic acid was added to the supernatant, and the mixture was acidified by standing at 4 °C for 30 min and then centrifuged. The supernatant was pipetted and filtered through a 0.22 µm filter for later use. A gas chromatograph was used to determine the short-chain fatty acid (SCFA) content in each filtered solution. The relevant parameters for the gas chromatography were as follows: instrument model: Agilent 7890A; chromatographic column model: DB-WAX UI (30 m × 0.32 mm × 0.25 µm) capillary column; column temperature: the initial temperature was 80 °C that lasted for 0.5 min, then the temperature was raised at 5 °C/min to 180 °C that lasted for 1 min, and then the temperature was raised at 40 °C/min to 220 °C that lasted for 4 min; injection port temperature: 220 °C; detector temperature: 250 °C; carrier gas: hydrogen at 30 mL/min; air at 300 mL/min; makeup gas flow: nitrogen at 28.314 mL/min; split ratio: 10:1; and sample injection volume: 1 µL. Four replicate injections were performed for the same sample, the average value was calculated, and the acid production was calculated according to the following formula: actual acid production (ppm) of the strain = measured acid production value of the strain - basal value of the BF839 medium. The total SCFA production was the sum of the production of acetic acid, propionic acid, butyric acid, isovaleric acid, and valeric acid.

Results: As shown in Table 2, the control strain LGG, the bacterial composition, and the single strains thereof all had ability to produce acetic acid. Beyond acetic acid production, Pdist-1 additionally produced propionic acid and isovaleric acid. The total SCFA production of Bbifi-1, Pdist-1, and the bacterial composition was significantly superior to that of LGG, while the total SCFA production of Lsali-1, Eaviu-1, and Lferm-1 was comparable to that of LGG.

**Table 2. Summary of acid production of strains**

| **Sample name** | **SCFAs** | **Measured value of sample (ppm)** | | | | **Mean** | **Actual yield (ppm)** | **Total SCFA production (ppm)** |
|---|---|---|---|---|---|---|---|---|
| | | **1** | **2** | **3** | **4** | | | |
| **BF839 medium** | Acetic acid | 218.9 | 215.8 | 218.8 | 213.9 | 216.8 | / | / |
| | Propionic acid | 0.0 | 0.0 | 0.0 | 0 | 0.0 | / | |
| | Butyric acid | 0.0 | 0.0 | 0.0 | 0 | 0.0 | / | |
| | Isovaleric acid | 0.0 | 0.0 | 0.0 | 0 | 0.0 | / | |
| | Valeric acid | 0.0 | 0.0 | 0.0 | 0 | 0.0 | / | |
| **LGG** | Acetic acid | 309.6 | 299.7 | 285.9 | 290.7 | 296.5 | 79.7 | 79.7 |
| | Propionic acid | 0.0 | 0.0 | 0.0 | 0.0 | 0.0 | 0.0 | |
| | Butyric acid | 0.0 | 0.0 | 0.0 | 0.0 | 0.0 | 0.0 | |
| | Isovaleric acid | 0.0 | 0.0 | 0.0 | 0.0 | 0.0 | 0.0 | |
| | Valeric acid | 0.0 | 0.0 | 0.0 | 0.0 | 0.0 | 0.0 | |
| **Bbifi-1** | Acetic acid | 1582.8 | 1569.5 | 1615.2 | 1644.9 | 1603.1 | 1386.3 | 1386.3 |
| | Propionic acid | 0.0 | 0.0 | 0.0 | 0.0 | 0.0 | 0.0 | |
| | Butyric acid | 0.0 | 0.0 | 0.0 | 0.0 | 0.0 | 0.0 | |
| | Isovaleric acid | 0.0 | 0.0 | 0.0 | 0.0 | 0.0 | 0.0 | |
| | Valeric acid | 0.0 | 0.0 | 0.0 | 0.0 | 0.0 | 0.0 | |
| **Eaviu-1** | Acetic acid | 280.5 | 268.4 | 278.1 | 268.5 | 273.9 | 57.1 | 57.1 |
| | Propionic acid | 0.0 | 0.0 | 0.0 | 0.0 | 0.0 | 0.0 | |
| | Butyric acid | 0.0 | 0.0 | 0.0 | 0.0 | 0.0 | 0.0 | |
| | Isovaleric acid | 0.0 | 0.0 | 0.0 | 0.0 | 0.0 | 0.0 | |
| | Valeric acid | 0.0 | 0.0 | 0.0 | 0.0 | 0.0 | 0.0 | |
| **Lsali-1** | Acetic acid | 321.7 | 326.3 | 316.1 | 326.9 | 322.8 | 105.9 | 105.9 |
| | Propionic acid | 0.0 | 0.0 | 0.0 | 0.0 | 0.0 | 0.0 | |
| | Butyric acid | 0.0 | 0.0 | 0.0 | 0.0 | 0.0 | 0.0 | |
| | Isovaleric acid | 0.0 | 0.0 | 0.0 | 0.0 | 0.0 | 0.0 | |
| | Valeric acid | 0.0 | 0.0 | 0.0 | 0.0 | 0.0 | 0.0 | |
| **Lferm-1** | Acetic acid | 284.3 | 271.4 | 278.6 | 279.5 | 278.4 | 61.6 | 61.6 |
| | Propionic acid | 0.0 | 0.0 | 0.0 | 0.0 | 0.0 | 0.0 | |
| | Butyric acid | 0.0 | 0.0 | 0.0 | 0.0 | 0.0 | 0.0 | |
| | Isovaleric acid | 0.0 | 0.0 | 0.0 | 0.0 | 0.0 | 0.0 | |
| | Valeric acid | 0.0 | 0.0 | 0.0 | 0.0 | 0.0 | 0.0 | |
| **Pdist-1** | Acetic acid | 604.5 | 604.7 | 619.6 | 612.8 | 610.4 | 393.6 | 828.4 |
| | Propionic acid | 368.2 | 366.3 | 393.4 | 368.5 | 374.1 | 374.1 | |
| | Butyric acid | 0.0 | 0.0 | 0.0 | 0.0 | 0.0 | 0.0 | |
| | Isovaleric acid | 59.2 | 59.6 | 64.5 | 59.6 | 60.7 | 60.7 | |
| | Valeric acid | 0.0 | 0.0 | 0.0 | 0.0 | 0.0 | 0.0 | |
| **Bacterial composition** | Acetic acid | 569.5 | 566.5 | 532.2 | 575.8 | 561.0 | 344.2 | 344.2 |
| | Propionic acid | 0.0 | 0.0 | 0.0 | 0.0 | 0.0 | 0.0 | |
| | Butyric acid | 0.0 | 0.0 | 0.0 | 0.0 | 0.0 | 0.0 | |
| | Isovaleric acid | 0.0 | 0.0 | 0.0 | 0.0 | 0.0 | 0.0 | |
| | Valeric acid | 0.0 | 0.0 | 0.0 | 0.0 | 0.0 | 0.0 | |

### Example 7: Ability of Single Strains and Bacterial Composition to Inhibit Pathogenic Bacteria

In this example, 8 common types of pathogenic bacteria capable of causing diarrhea were selected for antibacterial ability detection. The source information of the pathogenic strains was as follows: *Pseudomonas aeruginosa* (CMCC(B)10104), purchased from National Institutes for Food and Drug Control, China; *Shigella dysenteriae* (CMCC(B)51252), purchased from National Institutes for Food and Drug Control, China; *Staphylococcus aureus* (CMCC(B)26003), purchased from National Institutes for Food and Drug Control, China; *Escherichia coli* (CMCC(B)44102), purchased from National Institutes for Food and Drug Control, China; *Salmonella paratyphi B* (CMCC(B)50094), purchased from National Institutes for Food and Drug Control, China; *Yersinia enterocolitica* CMCC(B)52204, purchased from National Institutes for Food and Drug Control, China; *Vibrio parahaemolyticus* (ATCC 17802), purchased from American Type Culture Collection; and *Clostridioides difficile* (CICC 22951), purchased from China Center of Industrial Culture Collection.

Single strains: The control strain LGG, and the single strains Pdist-1, Eaviu-1, Lferm-1, Bbifi-1, and Lsali-1 were each inoculated into a three-component mixed liquid medium at an inoculation amount of 5%, and then anaerobically cultured for 48 h to obtain a fermentation broth.

Bacterial composition: Pdist-1, Eaviu-1, Lferm-1, Bbifi-1, and Lsali-1 were mixed and inoculated into a three-component mixed liquid medium, with the inoculation amount of each strain being 1%. The mixture was anaerobically cultured for 48 h to obtain a fermentation broth.

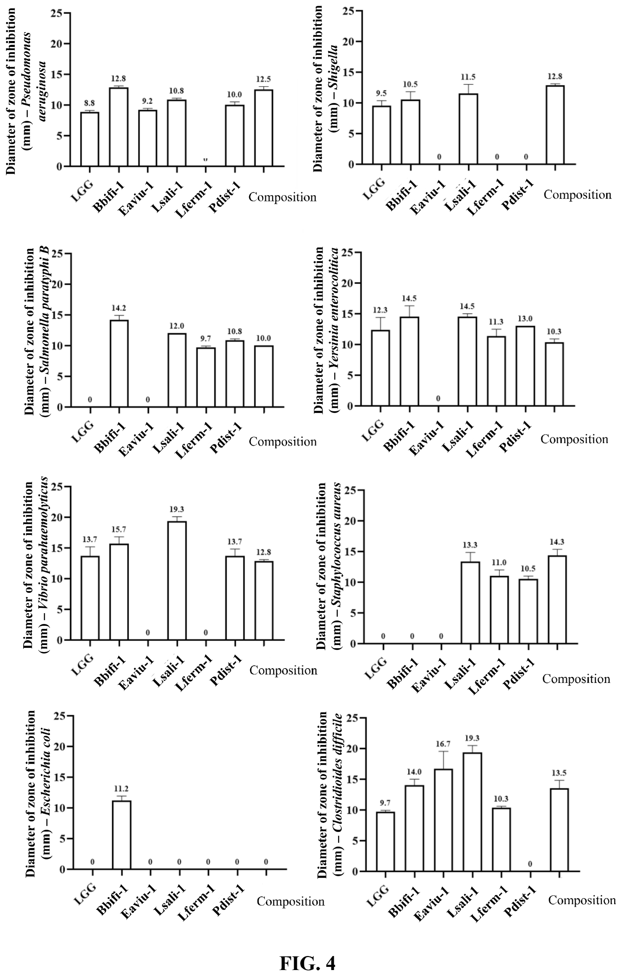

*Pseudomonas aeruginosa, Shigella dysenteriae, Staphylococcus aureus, Escherichia coli, Salmonella paratyphi B, Yersinia enterocolitica,* and *Vibrio parahaemolyticus* were each aerobically cultured in a TSB medium to the logarithmic growth phase. Each culture was diluted with a TSB medium to 10⁶ CFU/mL, and 200 µL of the dilution was spread onto a TSA solid medium. *Clostridioides difficile* was anaerobically cultured in a three-component mixed liquid medium to the logarithmic growth phase. The culture was diluted to 10⁶ CFU/mL with a three-component mixed liquid medium, and 200 µL of the dilution was spread onto an oxygen-free GAM solid medium (supplemented with 5% (v/v) horse serum, Beijing Solarbio Science & Technology Co., Ltd., S9050). Three Oxford cups were placed in each plate, and 200 µL of the fermentation broth to be tested (the aforementioned culture) was added into each Oxford cup. *Clostridioides difficile* was cultured under anaerobic conditions, while the other pathogenic bacteria were cultured under aerobic conditions. After the bacteria were cultured at 37 °C for 24 h, the diameters of the zones of inhibition were measured and the average values were calculated. The results are shown in FIG. 4. It can be seen that the bacterial composition exhibited an inhibitory effect on the growth of *Pseudomonas aeruginosa, Shigella dysenteriae, Salmonella paratyphi B, Yersinia enterocolitica, Vibrio parahaemolyticus, Staphylococcus aureus,* and *Clostridioides difficile.* The bacterial composition compensated for the deficiencies of single strains in inhibiting certain pathogenic bacteria. The bacterial composition demonstrated superior inhibitory activity against *Salmonella paratyphi B* and *Staphylococcus aureus* compared to the control strain LGG, while its inhibitory ability against the other pathogenic bacteria was comparable to that of the control strain LGG.

### Example 8: Test of Adhesion Ability of Single Strains and Bacterial Composition to Caco2 Cells

Single strains: The control strain LGG, and the single strains Pdist-1, Eaviu-1, Lferm-1, Bbifi-1, and Lsali-1 were each inoculated into a three-component mixed liquid medium at an initial inoculation amount of 2%, and then anaerobically cultured at 37 °C to the late logarithmic growth phase. Each cultured bacterial solution was centrifugally washed twice with sterile PBS (Wuhan Boster Biological Technology Co., Ltd., PYG0021), and then the bacterial pellet was diluted to 5 × 10⁸ CFU/mL with a DMEM medium (Thermo Fisher Scientific (China) Co., Ltd., C11995500BT) containing 10% (v/v) FBS (Thermo Fisher Scientific (China) Co., Ltd., SH30084.03) for later use.

Bacterial composition: The diluted bacterial solutions of the single strains Pdist-1, Eaviu-1, Lferm-1, Bbifi-1, and Lsali-1 described above were mixed in equal volumes to give a bacterial composition.

Caco-2 cells (Shangcheng BeNa Culture Collection, 350769) are adherent cells. The Caco-2 cells were digested using a trypsin cell digestion solution (Labgic Technology Co., Ltd., BL501A) pre-warmed to 37 °C. The digested Caco-2 cells were collected by centrifugation. The pellet was diluted with a DMEM medium containing 10% (v/v) FBS. The Caco-2 cells were inoculated into a 96-well plate at a density of 5 × 10⁴ CFU/well and then cultured overnight in a carbon dioxide incubator at 37 °C for later use.

The wells of the 96-well plate were divided into two groups. For each group, 100 µL of each of the diluted suspensions of the single strains and the bacterial composition was added to the 96-well cell culture plate containing Caco-2 cells. After the sample addition was finished, the 96-well cell culture plate was placed in a horizontal centrifuge and centrifuged at 1000 g for 1 min. The wells corresponding to each bacterial solution were divided into two subgroups: one subgroup was incubated for 30 min, and the other subgroup was incubated for 2 h. After incubation, the plate was washed twice with sterile PBS to wash away unadhered bacterial cells. After washing, 50 µL of a 0.25% trypsin cell digestion solution (Labgic Technology Co., Ltd., BL501A) was added to each well, and the plate was placed in an incubator at 37 °C for cell digestion. After the Caco-2 cells were digested and presented in the form of spheres, 150 µL of a DMEM medium was added to each well, and the mixture was pipetted repeatedly for about 1 min. After the cells and strains were digested and separated as confirmed by microscopic examination, 20 µL of the above mixed solution was pipetted and serially diluted 10-fold with 0.1% Tween 80-PBS in the 96-well plate. A solution with an appropriate dilution gradient was poured into the dissolved three-component mixed solid medium, and the cells were cultured at 37 °C for 48 h and then counted.

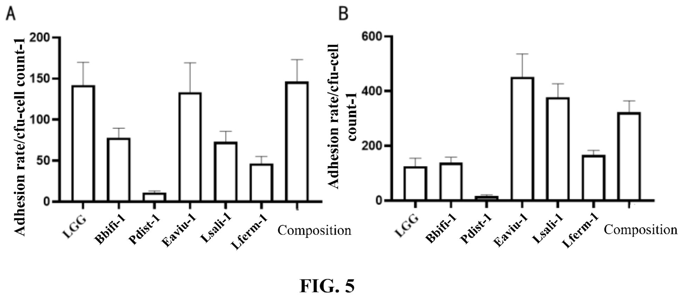

The results are shown in FIG. 5. Panel A showed that after the strains adhered to Caco2 cells for 30 min, the adhesion ability of *Enterococcus avium* Eaviu-1 and the bacterial composition was comparable to that of LGG. Panel B shows the adhesion effect of each strain after 2 h of adhesion time; the adhesion ability of *Bifidobacterium bifidum* Bbifi-1 and *Limosilactobacillus fermentum* Lferm-1 was comparable to that of LGG, while the adhesion ability of *Lactobacillus salivarius* Lsali-1, *Enterococcus avium* Eaviu-1, and the bacterial composition was superior to that of LGG, where the bacterial composition had better adhesion ability and thus was easier to colonize.

### Example 9: Test of Therapeutic Effect of Bacterial Composition on Mice with 5-Fluorouracil (5-FU)-Induced Diarrhea

### Preparation of bacterial composition:

Pdist-1, Lferm-1, Lsali-1, Eaviu-1, and Bbifi-1 were each inoculated into the corresponding bacterial powder preparation medium, and then anaerobically cultured at 37 °C and 90 rpm for 16-24 h to obtain a primary seed solution.

Subsequently, the five primary seed solutions were each transferred to the corresponding bacterial powder preparation medium, and then anaerobically cultured at 37 °C and 90 rpm for 10-15 h to obtain a secondary seed solution. The five secondary seed solutions were each pumped into a fermenter containing the corresponding bacterial powder preparation medium using a peristaltic pump, and then subjected to fermentation culture. After the fermentation was stopped, the bacterial cells of each strain were collected by centrifugation. Each corresponding lyoprotectant was added according to a weight ratio of the bacterial sludge to the lyoprotectant of 1:1 to 1:2, and the mixture was well mixed to emulsify the bacterial sludge. Each bacterial sludge was lyophilized and pulverized to obtain a bacterial powder. An appropriate amount of each pulverized bacterial powder was taken for determination of viable count. The bacterial powders of the strains were mixed at equal CFU concentrations based on the viable counts to obtain a bacterial composition powder. Finally, the bacterial composition powder was prepared into a bacterial suspension using normal saline for an animal experiment. The bacterial suspension was diluted with normal saline to obtain bacterial compositions with different bacterial doses.

### Experimental method:

(1) Experimental design: Ninety-six SPF-grade male Balb/c mice weighing 22-24 g (purchased from Beijing Vital River Laboratory Animal Technology Co., Ltd.) were housed in an SPF-grade animal room. Based on the initial body weight, the mice were randomly divided into 8 groups of 12: a normal control group, a model control group, a positive control loperamide group, a positive control Zhengchangsheng group, a high-dose bacterial composition group, a medium-high-dose bacterial composition group, a medium-dose bacterial composition group, and a low-dose bacterial composition group. The aforementioned 8 groups were intragastrically given normal saline (0.2 mL/mouse/day), lyoprotectant (0.2 mL/mouse/day), loperamide (purchased from Henan Zhongjie Pharmaceutical Co., Ltd., 20 mg/kg body weight/mouse/day), Zhengchangsheng (containing viable *Bacillus licheniformis,* Northeast Pharmaceutical Group Co., Ltd., 2 × 10⁸ CFU/mouse/day), high-dose bacterial composition (1 × 10⁹ CFU/mouse/day), medium-high-dose bacterial composition (1 × 10⁸ CFU/mouse/day), medium-dose bacterial composition (1 × 10⁷ CFU/mouse/day), and low-dose bacterial composition (1 × 10⁶ CFU/mouse/day), respectively. The overall experimental period was 9 days, designated as D1 to D9. Intragastric administration was performed for 8 consecutive days from D1 to D8. Modeling was started on D3. The mice in the normal control group received abdominal injection of normal saline at 0.2 mL/mouse, while the mice in all other groups were intraperitoneally injected with 5-FU (5-fluorouracil, purchased from Tianjin Pharma Heping (Tianjin) Pharmaceutical Co., Ltd.; specification: 10 mL/vial, 0.25 g/10 mL) at 30 mg/kg body weight for 4 consecutive days to induce a CID mouse model. The mice were dissected on D9. The specific experimental grouping and administration regimens are shown in Table 3.

**Table 3. Experimental grouping and administration regimens for bacterial composition in treating mice with 5-FU-induced diarrhea**

| **Group** | **Number of animals (mice)** | **Modeling agent** | **Modeling dose** | **Dose of administration** | **Volume of administration** | **Route of administration** | **Frequency of administration** |
|---|---|---|---|---|---|---|---|
| Normal control group | 12 | Normal saline | / | Normal saline | 0.2 mL/mouse | i.g. | QD×8 |
| Model control group | 12 | 5-FU | 30 mg/kg, i.p QD×4d | Lyoprotectant | 0.2 mL/mouse | i.g. | QD×8 |
| Loperamide group | 12 | 5-FU | 30 mg/kg, i.p QD×4d | 20 mg/kg | 20 mL/kg | i.g. | QD×8 |
| Zhengchangsheng group | 12 | 5-FU | 30 mg/kg, i.p QD×4d | 2 × 10⁸ CFU/mouse/day | 0.4 mL/mouse | i.g. | QD×8 |
| High-dose bacterial composition group | 12 | 5-FU | 30 mg/kg, i.p QD×4d | 1 × 10⁹ CFU/mouse/day | 0.2 mL/mouse | i.g. | QD×8 |
| Medium-high-dose bacterial composition group | 12 | 5-FU | 30 mg/kg, i.p QD×4d | 1 × 10⁸ CFU/mouse/day | 0.2 mL/mouse | i.g. | QD×8 |
| Medium-dose bacterial composition group | 12 | 5-FU | 30 mg/kg, i.p QD×4d | 1 × 10⁷ CFU/mouse/day | 0.2 mL/mouse | i.g. | QD×8 |
| Low-dose bacterial composition group | 12 | 5-FU | 30 mg/kg, i.p QD×4d | 1 × 10⁶ CFU/mouse/day | 0.2 mL/mouse | i.g. | QD×8 |

| | | | | | | | |
|---|---|---|---|---|---|---|---|
| Note: 5-FU indicates 5-fluorouracil; CFU indicates colony forming unit; d indicates days; i.p. indicates intraperitoneal injection; i.g. indicates intragastric administration; and QD indicates once daily. | | | | | | | |

During the experimental period, the body weight of the animals was measured daily, and general observations were recorded (the observation items included but were not limited to the animals' appearance signs, behaviors and activities, respiration, glandular secretion, fecal condition, etc.). The diarrhea condition of each animal after modeling was specifically observed and then recorded and scored. After euthanasia was performed at the end of the experiment, middle colon tissue samples were collected for qPCR detection of genes related to inflammatory factors (*TNF-α* and *IL-1β*) and tight junction proteins (*ZO-1* and *Occludin).*
(2) Diarrhea observation and scoring: The diarrhea scoring criteria followed the diarrhea scoring method in the study by Kurita A et al. (Modified irinotecan hydrochloride (CPT-11) administration schedule improves induction of delayed-onset diarrhea in rats. Kurita A et al., Cancer Chemother Pharmacol. 2000, 46(3):211-20). The mice were placed in mouse cages with clean filter paper at the bottom, with 1 mouse per cage. 0 points: hard, normal feces; 1 point: mildly wet feces, slightly wet feces, or soft feces; 2 points: moderately wet feces, shapeless feces, and perianal uncleanliness; and 3 points: severe diarrhea with loose feces, watery feces, and severe perianal uncleanliness. During the experiment, the feces of the mice were observed and scored daily. The total diarrhea score is the sum of daily diarrhea scores.
(3) qPCR detection: The total RNA was extracted from colon tissues of the mice in each group using the Trizol method and then reverse-transcribed into cDNA. The cDNA was stored at -20 °C for later use. The relative mRNA transcriptional levels of the genes encoding the pro-inflammatory factors *IL-1β* and *TNF-α,* as well as the tight junction proteins *ZO-1* and *Occludin-1,* in the colon of the mice in each group were detected using the qRT-PCR method (the primer sequences are shown in Table 7, and the internal reference gene was *β-actin*)*.* Reaction program: 1) 95 °C for 3 min; 2) 95 °C for 10 s, 60 °C for 30 s, 95 °C for 15 s, and 60 °C for 1 min, 40 cycles in total; and 3) 95 °C for 10 s. Data analysis was performed using the 2^{-ΔΔCT} method.
(4) Data statistics and analysis: The data for body weight, diarrhea scores, pathological examination results, etc., were expressed as mean ± standard error of the mean (Mean ± SEM). One-way ANOVA statistical analysis was performed using SPSS statistical software 26.0.

### Experimental results:

(1) The diarrhea scoring results are shown in Table 4.

**Table 4. Diarrhea scoring results**

| **Group** | **Dose of administration** | **D8 diarrhea score** | **D9 diarrhea score** | **Total diarrhea score** |
|---|---|---|---|---|
| Normal control group | / | 0.00±0.00**** | 0.00±0.00**** | 0.00+0.00**** |
| Model control group | / | 2.58+0.15 | 2.33±0.14 | 6.67+0.47 |
| Loperamide group | 20 mg/kg | 0.08±0.08**** | 0.17±0.11**** | 0.25±0.18**** |
| Zhengchangsheng group | 2 × 10⁸ CFU/mouse/day | 1.50±0.26**** | 1.00±0.12**** | 3.58±0.58**** |
| High-dose bacterial composition group | 1 × 10⁹ CFU/mouse/day | 1.33±0.22**** | 0.83±0.17**** | 3.50±0.51**** |
| Medium-high-dose bacterial composition group | 1 × 10⁸ CFU/mouse/day | 1.67±0.22*** | 1.42±0.19**** | 4.42±0.47*** |
| Medium-dose bacterial composition group | 1 × 10⁷ CFU/mouse/day | 1.58±0.15*** | 1.25±0.18**** | 4.17±0.34**** |
| Low-dose bacterial composition group | 1 × 10⁶ CFU/mouse/day | 1.58+0.19*** | 1.25±0.13**** | 4.50±0.42*** |

| | | | | |
|---|---|---|---|---|
| Note: All data were expressed as mean (Mean) ± standard error of the mean (SEM); for all groups compared with the model control group, "*" indicates P < 0.05, "**" indicates P < 0.01, "***" indicates P < 0.001, and "****" indicates P < 0.0001. | | | | |

After 5-FU induction, the D8 and D9 diarrhea scores of the model group were significantly higher than those of the normal control group. All 4 doses of the bacterial composition could significantly improve the diarrhea condition of the animals, with significantly lower D8 and D9 diarrhea scores and total diarrhea scores compared with the model control group. It can be seen that the bacterial composition of the present disclosure was able to ameliorate 5-FU-induced diarrhea.
(2) The qPCR detection results are shown in Table 5.

**Table 5. Effect of bacterial composition on expression of inflammation and intestinal barrier-related genes in colon tissues of mice with 5-FU-induced diarrhea**

| **Category** | **Gene** | **Normal control group** | **Model control group** | **Loperamide group** | **Zhengchangsheng group** | **High-dose bacterial composition group** | **Medium-high-dose bacterial composition group** | **Medium-dose bacterial composition group** | **Low-dose bacterial composition group** |
|---|---|---|---|---|---|---|---|---|---|
| Inflammatory factor | *TNF-a* | 1.00±0.16** | 1.98±0.28 | 1.05±0.21** | 2.16±0.20 | 1.35±0.18* | 1.40±0.19 | 1.45±0.19 | 1.61±0.23 |
| | *IL-1β* | 1.00±0.27** | 1.68±0.26 | 1.26±0.22 | 1.98±0.23 | 1.08±0.18* | 0.98±0.22* | 1.03±0.15* | 1.27±0.13 |
| Tight junction protein | *ZO-1* | 1.00±0.10*** | 0.58±0.08 | 0.80±0.08* | 0.94±0.08*** | 0.77±0.06* | 0.67±0.04 | 0.78±0.04* | 0.80±0.06* |
| | *Occl udin* | 1.00±0.20* | 0.61±0.07 | 0.90±0.10 | 1.29±0.13** | 0.95±0.08 | 1.09±0.10* | 1.03±0.22* | 0.83±0.08 |

| | | | | | | | | | |
|---|---|---|---|---|---|---|---|---|---|
| Note: All data were expressed as mean (Mean) ± standard error of the mean (SEM); for all groups compared with the model control group, "*" indicates P < 0.05, "**" indicates P < 0.01, "***" indicates P < 0.001, and "****" indicates P < 0.0001. | | | | | | | | | |

In the model control group, the relative mRNA transcription levels of the *TNF-α* and *IL-1β* genes were significantly increased, while the relative mRNA transcription levels of the *ZO-1* and *Occludin* genes were significantly reduced, as compared with the normal control group. The high-dose bacterial composition and loperamide could significantly reduce the relative mRNA transcription level of *TNF-α.* The bacterial composition at high dose, medium-high dose, and medium dose could significantly reduce the relative mRNA transcription level of *IL-1β.* The high-dose group, medium-dose group, and low-dose group could significantly increase the relative mRNA transcription level of *ZO-1*, showing amelioration effects comparable to those of loperamide and Zhengchangsheng. The bacterial composition at medium-high dose and medium dose could significantly increase the relative mRNA transcription level of *Occludin,* showing amelioration effects comparable to that of Zhengchangsheng.

In conclusion, the bacterial composition of the present disclosure was able to significantly reduce the diarrhea scores in the 5-FU-induced CID mouse model, and could demonstrate a significant therapeutic effect on diarrhea by reducing inflammatory factors and increasing the expression levels of tight junction proteins.

### Example 10: Therapeutic Effect of Bacterial Composition on Mice with Irinotecan (CPT-11)-Induced Diarrhea

The bacterial composition used in Examples 10-12 were obtained by mixing five strains at equal CFU concentrations according to the method in Example 9. The CFU of the bacterial composition referred to in Examples 10-12 was the total CFU of the five strains.

### Experimental method:

(1) Experimental design: Seventy male Balb/c mice (purchased from Shanghai Jihui Laboratory Animal Care Co., Ltd.) were housed in an SPF-grade animal room. Based on the body weight, the mice were randomly divided into 7 groups of 10: a normal control group (no administration), a model control group (administration regimen: lyoprotectant at 0.2 mL/mouse/day), a positive control Zhengchangsheng group (administration regimen: *Bacillus licheniformis* at 2.5 × 10⁸ CFU/mouse/day), a high-dose bacterial composition group (administration regimen: bacterial composition at 1 × 10⁹ CFU/mouse/day), a medium-high-dose bacterial composition group (administration regimen: bacterial composition at 1 × 10⁸ CFU/mouse/day), a medium-dose bacterial composition group (administration regimen: bacterial composition at 1 × 10⁷ CFU/mouse/day), and a low-dose bacterial composition group (administration regimen: bacterial composition at 1 × 10⁶ CFU/mouse/day). The overall experimental period was 10 days, designated as D1 to D10.

The mice in the normal control group received abdominal injection of normal saline at 0.2 mL/mouse, while the mice in all other groups received abdominal injection of CPT-11 at 85 mg/kg body weight for modeling. The modeling was started on D3 and performed for 4 consecutive days from D3 to D6, with one injection administered daily. The corresponding groups were each subjected to intragastric administration once daily for 9 consecutive days, and dissected on D10. The specific experimental grouping and administration regimens are shown in Table 6.

**Table 6. Experimental grouping and administration regimens for bacterial composition in treating mice with CPT-11-induced diarrhea**

| **Group** | **Number of animals (mice)** | **Modeling agent** | **Modeling dose** | **Dose of administration** | **Volume of administration** | **Route of administration** | **Frequency of administration** |
|---|---|---|---|---|---|---|---|
| Normal control group | 10 | Normal saline | / | / | 0.2 mL/mouse | i.g. | QD×9 |
| Model control group | 10 | CPT-11 | 85 mg/kg, i.p; QD×4d | Lyoprotectant | 0.2 mL/mouse | i.g. | QD×9 |
| Zhengchangsheng group | 10 | CPT-11 | 85 mg/kg, i.p; QD×4d | 2.5 × 10⁸ CFU/mouse/day | 0.4 mL/mouse | i.g. | QD×9 |
| High-dose bacterial composition group | 10 | CPT-11 | 85 mg/kg, i.p; QD×4d | 1 × 10⁹ CFU/mouse/day | 0.2 mL/mouse | i.g. | QD×9 |
| Medium-high-dose bacterial composition group | 10 | CPT-11 | 85 mg/kg, i.p; QD×4d | 1 × 10⁸ CFU/mouse/day | 0.2 mL/mouse | i.g. | QD×9 |
| Medium-dose bacterial composition group | 10 | CPT-11 | 85 mg/kg, i.p; QD×4d | 1 × 10⁷ CFU/mouse/day | 0.2 mL/mouse | i.g. | QD×9 |
| Low-dose bacterial composition group | 10 | CPT-11 | 85 mg/kg, i.p; QD×4d | 1 × 10⁶ CFU/mouse/day | 0.2 mL/mouse | i.g. | QD×9 |

| | | | | | | | |
|---|---|---|---|---|---|---|---|
| Note: CPT-11 indicates irinotecan hydrochloride; CFU indicates colony forming unit; d indicates days; i.p. indicates intraperitoneal injection; i.g. indicates intragastric administration; and QD indicates once daily. | | | | | | | |

During the experimental period, the body weight of the animals was measured daily, and general observations were recorded (the observation items included but were not limited to the animals' appearance signs, behaviors and activities, respiration, glandular secretion, fecal condition, etc.). The diarrhea condition of each animal after modeling was specifically observed and then recorded and scored. At the end of the experiment, whole blood samples were collected from the abdominal vena cava of animals in each group and subjected to blood routine examination. After animal euthanasia, the intestinal tract was taken, and the small intestine length was measured; the spleen was taken and weighed, and the spleen coefficient was calculated (spleen coefficient = spleen weight/body weight × 100). Samples were collected near the cecum end from 5 mice in each group, then rapidly frozen in liquid nitrogen, and cryopreserved at -80 °C. The samples were used for qPCR detection of mRNA expression levels of related genes such as inflammatory factors (*TNF-a, IL-1β,* and *IL-22*)*,* tight junction proteins (*ZO-1* and *Occludin*)*,* pro-apoptotic factor (*Bax*)*,* and aquaporin (*AQP8*)*.* The colorectal parts of the remaining 5 mice in each group were directly fixed in a 10% formaldehyde solution, stained with HE, and then subjected to pathological examination.

(2) Diarrhea observation and scoring: The diarrhea scoring criteria and procedures were the same as in Example 9.

(3) Blood routine examination: Whole blood samples were collected from the abdominal vena cava of animals in each group. Blood routine examination was performed on the samples using an automatic hematology analyzer (Mindray).

(4) qPCR detection: The total RNA was extracted from colon tissues of the mice in each group using the Trizol method and then reverse-transcribed into cDNA. The cDNA was stored at -20 °C for later use. The relative mRNA transcription levels of the genes of the pro-inflammatory factors *TNF-α* and *IL-1β,* anti-inflammatory factor *IL-22,* tight junction proteins *ZO-1* and *Occludin,* pro-apoptotic factor *Bax,* and aquaporin *AQP8* in the colon of the mice in each group were detected using the qPCR method (the primer sequences are shown in Table 7, and the internal reference gene was *β-actin*)*.* Reaction program: 1) 95 °C for 3 min; 2) 95 °C for 10 s, 60 °C for 30 s, 95 °C for 15 s, and 60 °C for 1 min, 40 cycles in total; and 3) 95 °C for 10 s. Data analysis was performed using the 2^{-ΔΔCT} method.

**Table 7. qRT-PCR primer information**

| **Gene** | **Primer** |
|---|---|
| *β-actin* | Forward (SEQ ID NO.1): 5'-CTAAGGCCAACCGTGAAAAG-3' |
| | Reverse (SEQ ID NO.2): 5'-ACCAGAGGCATACAGGGACA-3' |
| *TNF-α* | Forward (SEQ ID NO.3): 5'-CTGTAGCCCACGTCGTAGC-3' |
| | Reverse (SEQ ID NO.4): 5'-TTGAGATCCATGCCGTTG-3' |
| *IL-1β* | Forward (SEQ ID NO.5): 5'-AGTTGACGGACCCCAAAAG-3' |
| | Reverse (SEQ ID NO.6): 5'-AGCTGGATGCTCTCATCAGG-3' |
| *IL-22* | Forward (SEQ ID NO.7): 5'-TGACGACCAGAACATCCAGA-3' |
| | Reverse (SEQ ID NO.8): 5'-AATCGCCTTGATCTCTCCAC-3' |
| *ZO-1* | Forward (SEQ ID NO.9): 5'-GATCATTCCACGCAGTCTCC-3' |
| | Reverse (SEQ ID NO.10): 5'-GGCCCCAGGTTTAGACATTC-3' |
| *Occludin* | Forward (SEQ ID NO.11): 5'-GCGATCATACCCAGAGTCTTTC-3' |
| | Reverse (SEQ ID NO.12): 5'-TGCCTGAAGTCATCCACACT-3' |
| *Bax* | Forward (SEQ ID NO.13): 5'-CTACAGGGTTTCATCCAGGATCGAGCA-3' |
| | Reverse (SEQ ID NO.14): 5'-CATGTCAGCTGCCACCCGGAA-3' |
| *AQP8* | Forward (SEQ ID NO.15): 5'-TCAAGACCATGCTGCTAATTCCC-3' |
| | Forward (SEQ ID NO.16): 5'-GGACTCACCACTTTAGCCA-3' |

(5) Pathological examination of intestinal tissues: The colorectal parts were directly fixed in a 10% formaldehyde solution, stained with HE, and then subjected to pathological examination.

(6) Data statistics and analysis: The data for body weight, diarrhea scores, pathological examination results, etc., were expressed as mean ± standard error of the mean (Mean ± SEM). One-way ANOVA statistical analysis was performed using SPSS statistical software 26.0.

### Experimental results:

(1) The mice in the model group exhibited obvious clinical disease-like manifestations (mainly including diarrhea, weight loss, intestinal atrophy, decreased spleen coefficient, and abnormal counts of peripheral blood lymphocytes and neutrophils) after induction by intraperitoneal injection of irinotecan, suggesting that the CID model was successfully constructed.

The diarrhea scoring results and body weight measurement results are shown in Table 8 and Table 9, respectively.

**Table 8. Effect of bacterial composition on diarrhea in CPT-11-induced CID model mice**

| **Group** | **D3 diarrhea score** | **D6 diarrhea score** | **D10 diarrhea score** | **D10 total diarrhea score** |
|---|---|---|---|---|
| Normal control group | 0.00±0.00 | 0.00±0.00 | 0.00±0.00 | 0.00±0.00**** |
| Model control group | 0.00±0.00 | 1.50±0.17 | 2.80±0.13 | 13.20±0.79 |
| Zhengchangsheng group | 0.00±0.00 | 1.20±0.13 | 2.20±0.20 | 10.70±0.67* |
| High-dose bacterial composition group | 0.00±0.00 | 1.30+0.15 | 2.20±0.25 | 10.60±0.83* |
| Medium-high-dose bacterial composition group | 0.00±0.00 | 1.20±0.20 | 2.40±0.22 | 11.20±1.07 |
| Medium-dose bacterial composition group | 0.00±0.00 | 1.30±0.21 | 2.60±0.16 | 12.20±0.65 |
| Low-dose bacterial composition group | 0.00±0.00 | 1.40±0.16 | 2.60±0.16 | 12.70±0.78 |

| | | | | |
|---|---|---|---|---|
| Note: All data were expressed as mean (Mean) ± standard error of the mean (SEM); for all groups compared with the model control group, "*" indicates P < 0.05, "**" indicates P < 0.01, "***" indicates P < 0.001, and "****" indicates P < 0.0001. | | | | |

The high-dose bacterial composition and Zhengchangsheng could significantly reduce the D10 total diarrhea score. The medium-high-dose bacterial composition showed a trend toward reducing the D10 total diarrhea score. As compared with the model control group, the reduction magnitude of the D10 total diarrhea score in the high-dose bacterial composition group was slightly higher than that in the positive control Zhengchangsheng group. Overall, the bacterial composition significantly ameliorated the diarrhea condition in the mice with CPT-II-induced diarrhea and showed a dose-dependent trend.

**Table 9. Effect of bacterial composition on weight loss in mice with CPT-11-induced diarrhea**

| **Group** | **D1 body weight (g)** | **D3 body weight (g)** | **D6 body weight (g)** | **D10 body weight (g)** | **D6 body weight change rate (D6 vs D1, %)** | **D10 body weight change rate (D10 vs D1, %)** |
|---|---|---|---|---|---|---|
| Normal control group | 22.85±0.27 | 23.17±0.21 | 23.79±0.30 | 22.18±0.30 | 4.13±0.74**** | -2.88±1.15**** |
| Model control group | 22.70±0.24 | 23.15±0.23 | 21.14±0.27 | 16.35±0.29 | -6.90±0.57 | -27.98±1.04 |
| Zhengchangsheng group | 22.83±0.28 | 23.37±0.31 | 21.58±0.24 | 19.28±0.41 | -5.43±0.79 | - 1 5.46±1.83**** |
| High-dose bacterial composition group | 22.87±0.29 | 23.32±0.37 | 21.80±0.42 | 19.33±0.64 | -4.69±1.37 | -15.51±2.58**** |
| Medium-high-dose bacterial composition group | 22.73±0.23 | 23.38±0.28 | 21.61±0.23 | 19.72±0.30 | -4.91±0.92 | -13.14±1.61**** |
| Medium-dose bacterial composition group | 22.67±0.24 | 23.37±0.23 | 20.92±0.36 | 18.13±0.34 | -7.74±1.24 | -19.96±1.56* |
| Low-dose bacterial composition group | 22.86±0.24 | 23.09±0.22 | 21.13±0.41 | 17.87±0.52 | -7.61±1.41 | -21.85±2.01 |

| | | | | | | |
|---|---|---|---|---|---|---|
| Note: All data were expressed as mean (Mean) ± standard error of the mean (SEM); for all groups compared with the model control group, "*" indicates P < 0.05, "**" indicates P < 0.01, "***" indicates P < 0.001, and "****" indicates P < 0.0001. | | | | | | |

The body weight of the model mice continuously decreased after modeling until the end point of the experiment. The bacterial composition at high dose, medium-high dose, and medium dose, and Zhengchangsheng could significantly alleviate D10 weight loss in the model mice, and the extent of alleviation in the high-dose bacterial composition treatment group was comparable to that in the Zhengchangsheng group.

(2) The intestinal and splenic results are shown in Table 10.

**Table 10. Effect of bacterial composition on intestinal and splenic lesions in mice with CPT-II-induced diarrhea**

| **Group** | **Small intestine length (cm)** | **Small intestine thickness (cm)** | **Spleen coefficient** |
|---|---|---|---|
| Normal control group | 41.70±0.27**** | 0.59±0.01**** | 0.40±0.01*** |
| Model control group | 38.30±0.35 | 0.68±0.01 | 0.31±0.01 |
| Zhengchangsheng group | 40.35±0.44* | 0.64±0.01*** | 0.39±0.01** |
| High-dose bacterial composition group | 40.16±0.62* | 0.64±0.01*** | 0.36±0.01 |
| Medium-high-dose bacterial composition group | 40.26±0.38* | 0.63±0.01**** | 0.38±0.02** |
| Medium-dose bacterial composition group | 39.89±0.58 | 0.65±0.01** | 0.38±0.02* |
| Low-dose bacterial composition group | 40.63±0.63** | 0.66±0.01 | 0.38±0.02* |

| | | | |
|---|---|---|---|
| Note: All data were expressed as mean (Mean) ± standard error of the mean (SEM); for all groups compared with the model control group, "*" indicates P < 0.05, "**" indicates P < 0.01, "***" indicates P < 0.001, and "****" indicates P < 0.0001. | | | |

Small intestine atrophy was significantly ameliorated in the model mice of the high-dose, medium-high-dose, and low-dose bacterial composition groups and the Zhengchangsheng group. Small intestine swelling was also significantly ameliorated in the model mice of the high-dose, medium-high-dose, and medium-dose bacterial composition groups and the Zhengchangsheng group. The bacterial composition at medium-high dose, medium dose, and low dose, and Zhengchangsheng could significantly ameliorate spleen atrophy in the model mice.

(3) The results of blood routine examination are shown in Table 11.

The bacterial composition at high dose and medium-high dose could significantly increase the lymphocyte content and reduce the neutrophil content in the peripheral blood of the model mice. Zhengchangsheng showed a trend toward increasing the lymphocyte content in the peripheral blood of the model mice, and could significantly reduce the neutrophil content. In the peripheral blood of the model mice in the high-dose, medium-high-dose, medium-dose, and low-dose bacterial composition groups and the Zhengchangsheng group, the percentage of lymphocytes was significantly increased, while the percentage of neutrophils was significantly reduced.

**Table 11. Blood routine examination results for effect of bacterial composition on mice with CPT-II-induced diarrhea**

| **Group** | **Lymphocyte content (10⁹/L)** | **Neutrophi l content (10⁹/L)** | **Percentage of lymphocytes (%)** | **Percentage of neutrophils (%)** |
|---|---|---|---|---|
| Normal control group | 1.96±0.52* | 1.30±0.24* | 55.30±5.95*** | 42.42±6.14*** |
| Model control group | 0.64±0.05 | 3.69±0.65 | 16.36±3.45 | 81.72±3.24 |
| Zhengchangsheng group | 1.30±0.14 | 1.41±0.14* | 47.00±2.49** | 51.00±2.47** |
| High-dose group of formula bacteria | 2.37±0.59** | 1.24±0.19* | 62.24±5.07*** * | 35.28±4.68*** * |
| Medium-high-dose group of formula bacteria | 2.92±0.19** * | 1.39±0.19* | 66.34±2.86*** * | 31.06±2.83*** * |
| Medium-dose group of formula bacteria | 1.48±0.25 | 1.59±0.24 | 47.10±5.00** | 50.64±5.34** |
| Low-dose group of formula bacteria | 1.25±0.17 | 2.43±1.21 | 44.32±11.02** | 54.28±11.08* |

| | | | | |
|---|---|---|---|---|
| Note: All data were expressed as mean (Mean) ± standard error of the mean (SEM); for all groups compared with the model control group, "*" indicates P < 0.05, "**" indicates P < 0.01, "***" indicates P < 0.001, and "****" indicates P < 0.0001. | | | | |

### (4) The qPCR detection results are shown in Table 12.

**Table 12. Effect of bacterial composition on gene expression in colon tissues of mice with CPT-II-induced diarrhea**

| **Category** | **Gene** | **Normal control group** | **Model control group** | **Zhengchangsheng group** | **High-dose bacterial composition group** | **Medium-high-dose bacterial composition group** | **Medium-dose bacterial composition group** | **Low-dose bacterial composition group** |
|---|---|---|---|---|---|---|---|---|
| Inflammatory factor | *TNF-a* | 1.00±0.36** | 4.90±0.87 | 2.77±0.43 * | 2.31±0.55** | 2.78±0.62* | 4.25±0.70 | 4.49±0.59 |
| | *IL-1β* | 1.00±0.24** | 3.17±0.46 | 1.74±0.39 | 1.07±0.13** | 2.42±0.72 | 4.73±0.67 | 4.40±0.65 |
| | *IL-22* | 1.00±0.34* | 5.48±2.15 | 2.49±0.71 | 1.63±0.52 | 3.98±1.65 | 6.44±2.09 | 6.93±4.92 |
| Tight junction protein | *ZO-1* | 1.00±0.11** | 0.64±0.07 | 0.76±0.05 | 0.71±0.05 | 0.86±0.05 | 0.94±0.13* | 1.27±0.11** |
| | *Occludin* | 1.00±0.05** | 0.50±0.14 | 0.67±0.08 | 0.60±0.05 | 1.02±0.17** | 0.80±0.16 | 1.16±0.10** |
| Apoptotic factor | *Bax* | 1.00±0.08** | 2.71±0.25 | 2.11±0.07 | 1.62±0.28* | 2.09±0.27 | 2.59±0.37 | 2.34±0.57 |
| Aquaporin | *AQP8* | 1.00±0.18* | 0.32±0.13 | 0.44±0.15 | 1.11±0.42* | 0.50±0.17 | 0.14±0.07 | 0.12±0.05 |

| | | | | | | | | |
|---|---|---|---|---|---|---|---|---|
| Note: All data were expressed as mean (Mean) ± standard error of the mean (SEM); for all groups compared with the model control group, "*" indicates P < 0.05, and "**" indicates P < 0.01. | | | | | | | | |

The mRNA expression of the inflammatory factor *TNF-a* in the colorectal parts of the model mice in the high-dose and medium-high-dose bacterial composition groups and the Zhengchangsheng group was significantly down-regulated; the high-dose bacterial composition could significantly down-regulate the mRNA expression of the inflammatory factor *IL-1β,* while the medium-high-dose bacterial composition group and the Zhengchangsheng group showed a certain down-regulation trend compared with the model group. The high-dose bacterial composition group and the Zhengchangsheng group showed a trend toward reduced mRNA expression level of *IL-22.* The expression of the tight junction protein-related gene *ZO-1* was significantly up-regulated in the medium-dose and low-dose bacterial composition groups, while the expression of the tight junction protein-related gene *Occludin* was significantly up-regulated in the medium-high-dose and low-dose bacterial composition groups. In the model mice of the high-dose bacterial composition group, the mRNA expression of the apoptosis-related gene *Bax* was significantly reduced, while the expression of aquaporin *AQP8* was significantly enhanced.

(5) The pathological examination results are shown in Table 13.

**Table 13. Effect of bacterial composition on colorectal pathological scores of mice with CPT-II-induced diarrhea**

| **Group** | **Inflammation** | **Damage** | **Crypt abscess** | **Muscularis propria thickness** | **Goblet cell count** | **Pathological score** |
|---|---|---|---|---|---|---|
| Normal control group | 0.00±0.00 | 0.00±0.00 | 0.00±0.00 | 0.00±0.00 | 0.00±0.00 | 0.00±0.00**** |
| Model control group | 2.40±0.24 | 2.00±0.45 | 0.80±0.20 | 1.00±0.00 | 1.00±0.00 | 7.20±0.80 |
| Zhengchangsheng group | 0.80±0.20 | 0.40±0.24 | 0.00±0.00 | 0.20±0.20 | 0.40±0.24 | 1.80±0.73* |
| High-dose bacterial composition group | 1.00±0.00 | 0.60±0.24 | 0.00±0.00 | 0.60±0.24 | 0.40±0.24 | 2.60±0.68 |
| Medium-high-dose bacterial composition group | 0.60±0.24* | 0.20±0.20 | 0.20±0.20 | 0.20±0.20 | 0.20±0.20 | 1.40±0.93** |
| Medium-dose bacterial composition group | 2.20±0.49 | 2.20±0.49 | 0.80±0.20 | 0.40±0.24 | 1.00±0.00 | 6.60±0.87 |
| Low-dose bacterial composition group | 0.60±0.24 | 0.20±0.20 | 0.00±0.00 | 0.20±0.20 | 0.20±0.20 | 1.20±0.73** |

| | | | | | | |
|---|---|---|---|---|---|---|
| Note: All data were expressed as mean (Mean) ± standard error of the mean (SEM); for all groups compared with the model control group, "*" indicates P < 0.05, "**" indicates P < 0.01, and "****" indicates P < 0.0001. | | | | | | |

The pathological scores of the medium-high-dose and low-dose bacterial composition groups and the Zhengchangsheng group were significantly lower than those of the model control group. The pathological scores of the high-dose bacterial composition group and the Zhengchangsheng group showed a reduction trend compared with the model group.

In conclusion, the bacterial composition could reduce CPT-11-induced diarrhea, decrease the mRNA expression of intestinal pro-inflammatory factors (*TNF-a*, *IL-1β,* and *IL-22*) and pro-apoptotic factor (*Bax*), and enhance the mRNA expression of tight junction proteins (*ZO-1* and *Occludin*) and aquaporin (*AQP8*), thereby significantly ameliorating symptoms such as weight loss, small intestine swelling, spleen atrophy, abnormal counts of peripheral blood immune cells (lymphocytes and neutrophils), and intestinal inflammation in mice with CPT-11-induced diarrhea.

### Example 11: Therapeutic Effect of Bacterial Composition on Mice with Radiation Enteritis

(1) Experimental design: Eighty male C57BL/6 mice (purchased from Chengdu GemPharmatech Co., Ltd.) were housed in an SPF-grade animal room. Based on the body weight, the aforementioned mice were randomly divided into 8 groups of 10: a normal control group (administration regimen: normal saline at 0.2 mL/mouse/day), a model control group (administration regimen: lyoprotectant at 0.2 mL/mouse/day), a loperamide group (administration regimen: loperamide at 15 mg/kg body weight), an LGG (*Lactobacillus rhamnosus* GG, Shaanxi Zelang Biotechnology Co., Ltd.) group (administration regimen: LGG at 1 × 10⁹ CFU/mouse/day), a high-dose bacterial composition group (administration regimen: bacterial composition at 1 × 10⁹ CFU/mouse/day), a medium-high-dose bacterial composition group (administration regimen: bacterial composition at 1 × 10⁸ CFU/mouse/day), a medium-dose bacterial composition group (administration regimen: bacterial composition at 1 × 10⁷ CFU/mouse/day), and a low-dose bacterial composition group (administration regimen: bacterial composition at 1 × 10⁶ CFU/mouse/day). Except for the normal control group, all other groups of mice were subjected to a single whole abdominal X-ray irradiation at a dose of 11.5 Gy. The overall experimental period was 18 days, designated as D1 to D18. Each group was subjected to intragastric administration once daily for 17 consecutive days. Seven days after administration, irradiation was performed on D8, and dissection was conducted on D18. The experimental grouping and administration regimens are shown in Table 14.

**Table 14. Experimental grouping and administration regimens for bacterial composition in treating mice with radiation enteritis**

| **Group** | **Number of animals (mice)** | **Molding** | **Dose of administration** | **Volume of administration** | **Route of administration** | **Frequency of administration** |
|---|---|---|---|---|---|---|
| Normal control group | 10 | / | Normal saline | 0.2 mL/mouse | i.g. | QD×17 |
| Model control group | 10 | Single whole abdominal X-ray irradiation at 11.5 Gy | Lyoprotectant | 0.2 mL/mouse | i.g. | QD×17 |
| Loperamide group | 10 | | 15 mg/kg | 10ml/kg | i.g. | QD×17 |
| LGG group | 10 | | 1 × 10⁹ CFU/mouse/day | 0.2 mL/mouse | i.g. | QD×17 |
| High-dose bacterial composition group | 10 | | 1 × 10⁹ CFU/mouse/day | 0.2 mL/mouse | i.g. | QD×17 |
| Medium-high-dose bacterial composition group | 10 | | 1 × 10⁸ CFU/mouse/day | 0.2 mL/mouse | i.g. | QD×17 |
| Medium-dose bacterial composition group | 10 | | 1 × 10⁷ CFU/mouse/day | 0.2 mL/mouse | i.g. | QD×17 |
| Low-dose bacterial composition group | 10 | | 1 × 10⁶ CFU/mouse/day | 0.2 mL/mouse | i.g. | QD×17 |

| | | | | | | |
|---|---|---|---|---|---|---|
| Note: CFU indicates colony forming unit; d indicates days; i.p. indicates intraperitoneal injection; i.g. indicates intragastric administration; and QD indicates once daily. | | | | | | |

During the experimental period, the body weight of the animals was measured daily, and general observations were recorded (the observation items included but were not limited to the animals' appearance signs, behaviors and activities, respiration, glandular secretion, fecal condition, etc.). The diarrhea condition of each animal after irradiation modeling was specifically observed and then recorded and scored. At the end of the experiment, the animals were euthanized, and the colorectal parts were taken, directly fixed in a 10% formaldehyde solution, stained with HE, and then subjected to pathological examination.
(2) Diarrhea observation and scoring: The diarrhea scoring criteria and procedures were the same as in Example 9.
(3) Pathological examination of intestinal tissues: The colorectal parts were directly fixed in a 10% formaldehyde solution, stained with HE, and then subjected to pathological examination.
(4) Data statistics and analysis: The data for body weight, diarrhea scores, pathological examination results, etc., were expressed as mean ± standard error of the mean (Mean ± SEM). One-way ANOVA statistical analysis was performed using SPSS statistical software 26.0.

Experimental results: After abdominal irradiation, the animals in the model control group exhibited obvious symptoms such as diarrhea, weight loss, intestinal mucosal damage, and inflammatory infiltration, suggesting that the radiation enteritis mouse model was successfully constructed.
(1) The diarrhea results are shown in Table 15.

**Table 15. Effect of bacterial composition on diarrhea condition in radiation enteritis model mice**

| **Group** | **D17 total diarrhea score** |
|---|---|
| Normal control group | 0.00±0.00**** |
| Model control group | 12.10±0.48 |
| Loperamide group | 9.80+0.59* |
| LGG group | 6.40+0.88**** |
| High-dose bacterial composition group | 9.00+1.15** |
| Medium-high-dose bacterial composition group | 10.60±0.69 |
| Medium-dose bacterial composition group | 9.50±0.85* |
| Low-dose bacterial composition group | 4.70±0.56**** |

| | |
|---|---|
| Note: All data were expressed as mean (Mean) ± standard error of the mean (SEM); for all groups compared with the model control group, "*" indicates P < 0.05, "**" indicates P < 0.01, "***" indicates P < 0.001, "****" indicates P < 0.0001, and "/" indicates not applicable. | |

Compared with the model control group, the high-dose, medium-dose, and low-dose bacterial composition groups, the loperamide group, and the LGG group all exhibited significant improvements in the D17 total diarrhea scores of the mice. The D17 total diarrhea scores of the medium-high-dose bacterial composition group showed a reduction trend.
(2) The body weight results are shown in Table 16.

**Table 16. Effect of bacterial composition on body weight changes in radiation enteritis model mice**

| **Group** | **D1 body weight (g)** | **D14 body weight (g)** | **D17 body weight (g)** | **D14 body weight change rate (%)** | **D17 body weight change rate (%)** |
|---|---|---|---|---|---|
| Normal control group | 23.27±0.23 | 24.05±0.26 | 24.06±0.34 | 3.36±4.83**** | 3.41=15.73**** |
| Model control group | 23.24±0.26 | 19.33±0.30 | 20.55±0.12 | -16.83±4.63 | -11.60±-16.72 |
| Loperamide group | 23.11±0.20 | 19.79±0.14 | 20.92±0.16 | -14.37±-10.00 | -9.48+-6.38 |
| LGG group | 23.19±0.26 | 20.31±0.32 | 21.34±0.15 | -12.41±6.95* | -7.99±-13.31 |
| High-dose bacterial composition group | 23.01±0.12 | 20.54±0.34 | 20.98±0.18 | -10.74±56.18*** | -8.80±15.38 |
| Medium-high-dose bacterial composition group | 23.24±0.35 | 19.19±0.25 | 20.23±0.17 | -17.41±-9.55 | -12.95±-16.06 |
| Medium-dose bacterial composition group | 22.85±0.23 | 18.94±0.18 | 20.17±0.16 | -17.15±-7.44 | -11.74±-9.53 |
| Low-dose bacterial composition group | 23.03±0.16 | 20.67±0.23 | 21.41±0.18 | -10.20±1.21*** | -6.99±0.91** |

| | | | | | |
|---|---|---|---|---|---|
| Note: All data were expressed as mean (Mean) ± standard error of the mean (SEM); for all groups compared with the model control group, "*" indicates P < 0.05, "**" indicates P < 0.01, "***" indicates P < 0.001, and "****" indicates P < 0.0001. | | | | | |

Compared with the model control group, the high-dose and low-dose bacterial composition groups and the LGG group exhibited significant amelioration in the weight loss of the mice on D14. The low-dose bacterial composition could significantly ameliorate the weight loss of the model mice on D17. The bacterial composition at high dose and medium dose, loperamide, and LGG all showed a trend toward restoring the D17 body weight of the model mice.
(3) The colorectal pathological results are shown in Table 17.

**Table 17. Effect of bacterial composition on pathological scores of colorectal parts of radiation enteritis model mice**

| **Group** | **Mucosal damage** | **Goblet cell** | **Inflammatory infiltration** | **Pathological score** |
|---|---|---|---|---|
| Normal control group | 0.00±0.00*** | 0.00±0.00** | 0.20±0.06*** | 0.20±0.06**** |
| Model control group | 2.60±0.08 | 2.40±0.08 | 2.40±0.08 | 7.40±0.08 |
| Loperamide group | 1.60±0.08 | 1.60±0.08 | 2.00±0.00 | 5.20±0.15 |
| LGG group | 1.20±0.06 | 1.00±0.10 | 1.40±0.08 | 3.60±0.13** |
| High-dose bacterial composition group | 1.60±0.13 | 1.20±0.06 | 2.00±0.10 | 4.80±0.15* |
| Medium-high-dose bacterial composition group | 2.00±0.14 | 1.60±0.16 | 2.20±0.12 | 5.80±0.23 |
| Medium-dose bacterial composition group | 1.60±0.13 | 1.40±0.08 | 2.00±0.10 | 5.00±0.10 |
| Low-dose bacterial composition group | 0.80±0.06* | 0.60±0.08* | 1.20±0.06* | 2.60±0.08**** |

| | | | | |
|---|---|---|---|---|
| Note: All data were expressed as mean (Mean) ± standard error of the mean (SEM); for all groups compared with the model control group, "*" indicates P < 0.05, "**" indicates P < 0.01, "***" indicates P < 0.001, and "****" indicates P < 0.0001. | | | | |

Compared with the model control group, the high-dose and low-dose bacterial composition groups and the LGG group all exhibited significant improvements in pathological scores. The pathological scores of the colorectal parts of the medium-high-dose and medium-dose bacterial composition groups and the loperamide group showed an improvement trend.

In conclusion, the bacterial composition had a significant therapeutic effect on the mice with radiation enteritis induced by whole abdominal X-ray irradiation, and could ameliorate the diarrhea severity and weight loss, and alleviate the intestinal lesion degree.

### Example 12: Therapeutic Effect of Bacterial Composition on 5-FU-Induced Tumor-Bearing CID Mice

Experimental design: Fifty male BALB/c mice (purchased from Chengdu GemPharmatech Co., Ltd.) were housed in an SPF-grade animal room. CT26 cells (purchased from Beijing BeNa Culture Collection) were subcutaneously inoculated into the right flank of each mouse at a dose of 5 × 10⁶ cells/mouse in a volume of 0.1 mL. When the tumor volume reached about 100-150 mm³, 42 mice were selected for the subsequent experiment. Based on the tumor volume, the aforementioned 42 mice were randomly divided into 7 groups of 6. The aforementioned 7 groups were as follows: a normal control group (administration regimen: lyoprotectant at 0.2 mL/mouse/day), a model control group (administration regimen: lyoprotectant at 0.2 mL/mouse/day), a positive control loperamide group (administration regimen: loperamide at 20 mg/kg body weight), a Zhengchangsheng group (administration regimen: *Bacillus licheniformis* at 2 × 10⁸ CFU/mouse/day), a high-dose bacterial composition group (administration regimen: bacterial composition at 1 × 10⁹ CFU/mouse/day), a medium-dose bacterial composition group (administration regimen: bacterial composition at 1 × 10⁸ CFU/mouse/day), and a low-dose bacterial composition group (administration regimen: bacterial composition at 1 × 10⁷ CFU/mouse/day). The day of grouping was designated as day 1 (D1) of the experiment, and intragastric administration was started on day 1 (D1) and performed once daily (QD) for 9 consecutive days. On days 3, 4, 5, and 6 of administration, the normal control group was intraperitoneally injected with normal saline at 0.2 mL/mouse, while the mice in all other groups were intraperitoneally injected with 5-FU (50 mg/kg body weight, 10 mL/kg body weight) for modeling. Compared with the normal control group, the tumor volumes of mice in each group were reduced after 5-FU injection. The model control group exhibited severe diarrhea and weight loss, and caused significant abnormalities in blood routine examination indexes, suggesting that the model was successfully constructed.

The specific administration regimens are detailed in Table 18. After the end of the experiment, whole blood samples were collected from animals in each group through enucleation, and then subjected to blood routine examination using a five-classification blood cell analyzer for animals (Shenzhen Dymind Biotechnology Co., Ltd.).

**Table 18. Administration regimens for 5-FU-induced tumor-bearing CID model**

| **Group** | **Number of animals (mice)** | **Modeling agent** | **Modeling dose** | **Dose of administration** | **Volume of administration** | **Route of administration** | **Frequency of administration** |
|---|---|---|---|---|---|---|---|
| Normal control group | 6 | Normal saline | / | Lyoprotectant | 0.2 mL/mouse | i.g. | QD×9 |
| Model control group | 6 | 5-FU | 50 mg/kg, i.p QD×4d | Lyoprotectant | 0.2 mL/mouse | i.g. | QD×9 |
| Loperamide group | 6 | 5-FU | 50 mg/kg, i.p QD×4d | 20 mg/kg | 10 ml/kg | i.g. | QD×9 |
| Zhengchangsheng group | 6 | 5-FU | 50 mg/kg, i.p QD×4d | 2 × 10⁸ CFU/mouse/day | 0.4 mL/mouse | i.g. | QD×9 |
| High-dose bacterial composition group | 6 | 5-FU | 50 mg/kg, i.p QD×4d | 1 × 10⁹ CFU/mouse/day | 0.2 mL/mouse | i.g. | QD×9 |
| Medium-dose bacterial composition group | 6 | 5-FU | 50 mg/kg, i.p QD×4d | 1 × 10⁸ CFU/mouse/day | 0.2 mL/mouse | i.g. | QD×9 |
| Low-dose bacterial composition group | 6 | 5-FU | 50 mg/kg, i.p QD×4d | 1 × 10⁷ CFU/mouse/day | 0.2 mL/mouse | i.g. | QD×9 |

| | | | | | | | |
|---|---|---|---|---|---|---|---|
| Note: i.g. indicates oral gavage, and QD indicates once daily. | | | | | | | |

Experimental results: The diarrhea results are shown in Table 19.

**Table 19. Diarrhea scores of mice in each group**

| **Group** | **D6 diarrhea score** | **D7 diarrhea score** | **D8 diarrhea score** | **D9 diarrhea score** | **D1-D9 total diarrhea score** |
|---|---|---|---|---|---|
| Normal control group | 0.00±0.00** | 0.00±0.00** | 0.00±0.00** | 0.00±0.00** | 0.00±0.00** |
| Model control group | 0.83±0.31 | 1.67±0.21 | 2.17±0.31 | 2.33±0.21 | 7.00±0.86 |
| Loperamide group | 0.33±0.21 | 1.00±0.00 | 1.33±0.21** | 2.00±0.37 | 4.67±0.56* |
| Zhengchangsheng group | 1.33±0.33 | 1.83±0.17 | 2.00±0.00 | 2.17±0.17 | 7.33±0.49 |
| High-dose bacterial composition group | 0.50±0.22 | 1.50±0.22 | 1.83±0.17 | 1.67±0.21 | 5.50+0.72 |
| Medium-dose bacterial composition group | 0.83±0.17 | 1.50±0.22 | 1.83±0.17 | 1.83±0.31 | 6.00+0.73 |
| Low-dose bacterial composition group | 0.17±0.17 | 1.50±0.22 | 1.50±0.22* | 1.50±0.34* | 4.67±0.80* |

| | | | | | |
|---|---|---|---|---|---|
| Note: All data were expressed as mean (Mean) ± standard error of the mean (SEM); for the diarrhea scores of each group compared with the model control group, "*" indicates P < 0.05, and "**" indicates P < 0.01. | | | | | |

Compared with the normal control group, the model control group exhibited diarrhea starting from D6, and the diarrhea severity worsened over time. Compared with the model control group, the loperamide group showed a significant reduction in the D8 diarrhea score and the total diarrhea score; the low-dose bacterial composition significantly reduced the D8 diarrhea score, the D9 diarrhea score, and the total diarrhea score.

The results of blood routine examination are shown in Table 20.

**Table 20. Results of blood routine examination in 5-FU-induced tumor-bearing mouse model**

| **Group** | **Red blood cell count (10¹²/L)** | **Hematocrit (%)** | **White blood cell count (10⁹/L)** | **Lymphocyte count (10⁹/L)** | **Platelet count (10⁹/L)** |
|---|---|---|---|---|---|
| Normal control group | 9.77±0.13 | 45.93±0.66* | 8.99±0.93** | 5.55±0.27** | 497.50±40.53** |
| Model control group | 8.76±0.27 | 40.20±1.28 | 1.08±0.22 | 1.02±0.22 | 226.17±24.53 |
| Loperamide group | 9.39±0.23 | 43.17±0.99 | 1.24±0.30 | 1.18±0.29 | 216.67±21.53 |
| Zhengchangsheng group | 10.06±0.03* | 46.87±0.42* | 1.22±0.13 | 1.19±0.12 | 274.17±4.90 |
| High-dose bacterial composition group | 9.78±0.12 | 44.88±0.49 | 1.27±0.18 | 1.24±0.17 | 278.83±11.87 |
| Medium-dose bacterial composition group | 10.05±0.13* | 47.07±0.64* | 1.65±0.16 | 1.59±0.15 | 286.50±7.46 |
| Low-dose bacterial composition group | 10.44±0.19** | 48.45±0.74** | 2.03±0.28 | 1.99±0.28** | 317.33±15.79** |

| | | | | | |
|---|---|---|---|---|---|
| Note: All data were expressed as mean (Mean) ± standard error of the mean (SEM); for the blood routine examination data of each group compared with the model control group, "*" indicates P < 0.05, and "**" indicates P < 0.01. | | | | | |

Compared with the normal control group, the model control group showed significantly reduced hematocrit, white blood cell count, lymphocyte count, and platelet count. Compared with the model control group, the medium-dose and low-dose bacterial composition groups and the Zhengchangsheng group showed significantly increased red blood cell count and hematocrit, while the low-dose bacterial composition group exhibited increased lymphocyte count and platelet count.

In conclusion, all three doses of the bacterial composition showed a trend toward ameliorating diarrhea and improving blood routine examination indexes; the low-dose bacterial composition and loperamide could significantly ameliorate diarrhea; the low-dose bacterial composition had the ability to significantly improve blood routine examination indexes (lymphocyte count, red blood cell count, and platelet count).

It can be known from the general technical knowledge that the present disclosure can be implemented by other embodiments without departing from the spirit or essential features thereof. Therefore, the embodiments disclosed above are, in every respect, merely illustrative and not exclusive. All modifications within the scope of the present disclosure or within the scope equivalent to the present disclosure are encompassed in the present disclosure.

## Claims

1. A probiotic composition, comprising a fifth probiotic and any two, any three, or four of a first probiotic, a second probiotic, a third probiotic, and a fourth probiotic, wherein
the first probiotic is selected from *Bifidobacterium bifidum,* a progeny strain of the *Bifidobacterium bifidum,* a clonal strain of the *Bifidobacterium bifidum,* and a pure culture of the *Bifidobacterium bifidum*;
the second probiotic is selected from *Enterococcus avium,* a progeny strain of the *Enterococcus avium,* a clonal strain of the *Enterococcus avium,* and a pure culture of the *Enterococcus avium;*
the third probiotic is selected from *Lactobacillus salivarius,* a progeny strain of the *Lactobacillus salivarius,* a clonal strain of the *Lactobacillus salivarius,* and a pure culture of the *Lactobacillus salivarius*;
the fourth probiotic is selected from *Limosilactobacillus fermentum,* a progeny strain of the *Limosilactobacillus fermentum,* a clonal strain of the *Limosilactobacillus fermentum,* and a pure culture of the *Limosilactobacillus fermentum*; and
the fifth probiotic is selected from *Parabacteroides distasonis,* a progeny strain of the *Parabacteroides distasonis,* a clonal strain of the *Parabacteroides distasonis,* and a pure culture of the *Parabacteroides distasonis.*

2. The probiotic composition according to claim 1, wherein the *Bifidobacterium bifidum* has a microbial deposit number of CCTCC NO: M2023349;
the *Enterococcus avium* has a microbial deposit number of CCTCC NO: M2023350;
the *Lactobacillus salivarius* has a microbial deposit number of CCTCC NO: M2023348;
the *Limosilactobacillus fermentum* has a microbial deposit number of CCTCC NO: M2023352; and
the *Parabacteroides distasonis* has a microbial deposit number of CCTCC NO: M20222033.

3. A microecological composition, comprising the probiotic composition according to claim 1 or 2 as an active material.

4. The microecological composition according to claim 3, further comprising an auxiliary material, wherein the auxiliary material is selected from a lyoprotectant, a bacterial culture medium, a food additive, an acceptable carrier or auxiliary material in a healthcare product, and a pharmaceutically acceptable carrier or auxiliary material.

5. The microecological composition according to claim 3, wherein in the microecological composition, based on the viable count of bacterial cells, the content ratio of any two strains is 100 CFU:(1-10000) CFU.

6. Use of the probiotic composition according to claim 1 or 2 or the microecological composition according to any one of claims 3-5 in the preparation of a product for use alone or in combination with an additional microbial formulation and/or medicament to improve the health status of a subject, wherein
the improvement of the health status of the subject is selected from:
inhibiting proliferation of any one, any two, any three, any four, any five, any six, any seven, or eight of *Pseudomonas aeruginosa, Shigella dysenteriae, Staphylococcus aureus, Escherichia coli, Salmonella paratyphi B, Yersinia enterocolitica, Vibrio parahaemolyticus,* and *Clostridioides difficile* in a body cavity of the subject;
treating, preventing, and/or mitigating tissue damage, diseases, or sub-health status caused by any one, any two, any three, any four, any five, any six, any seven, or eight of *Pseudomonas aeruginosa, Shigella dysenteriae, Staphylococcus aureus, Escherichia coli, Salmonella paratyphi B, Yersinia enterocolitica, Vibrio parahaemolyticus,* and *Clostridioides difficile;*
improving the antioxidant capacity in the intestinal tract of the subject;
treating, preventing, and/or mitigating diarrhea caused by an anti-tumor drug;
treating, preventing, and/or mitigating intestinal inflammation caused by an anti-tumor drug;
treating, preventing, and/or mitigating weight loss caused by an anti-tumor drug;
treating, preventing, and/or mitigating shortening of small intestine length caused by an anti-tumor drug;
treating, preventing, and/or mitigating an increase in small intestine thickness caused by an anti-tumor drug;
treating, preventing, and/or mitigating intestinal injury caused by an anti-tumor drug;
treating, preventing, and/or mitigating a decrease in spleen-to-body weight ratio caused by an anti-tumor drug;
treating, preventing, and/or mitigating tissue damage, diseases, or sub-health status caused by increased expression levels of any one, any two, any three, or four of TNF-α, IL-1β, IL-22, and Bax;
treating, preventing, and/or mitigating tissue damage, diseases, or sub-health status caused by decreased expression levels of any one, any two, or three of ZO-1, Occludin, and AQP8;
treating, preventing, and/or mitigating diarrhea caused by radiotherapy;
treating, preventing, and/or mitigating weight loss caused by radiotherapy;
treating, preventing, and/or mitigating intestinal injury caused by radiotherapy;
treating, preventing, and/or mitigating intestinal inflammation caused by radiotherapy; and
treating, preventing, and/or mitigating a decrease in hematocrit, a decrease in white blood cell count, a decrease in lymphocyte count, and/or a decrease in platelet count caused by an anti-tumor drug.

7. The use according to claim 6, wherein the product is a food, a healthcare product, or a pharmaceutical product.

8. The use according to claim 6, wherein the subject is selected from a human and a mouse.

9. The use according to claim 6, wherein the anti-tumor drug is selected from a chemotherapeutic drug, a targeted drug, and an immune checkpoint inhibitor.

10. The use according to claim 9, wherein the anti-tumor drug is selected from epirubicin, actinomycin D, doxorubicin, daunorubicin, paclitaxel, docetaxel, albumin-bound paclitaxel, cisplatin, carboplatin, nedaplatin, oxaliplatin, lobaplatin, cyclophosphamide, nitrogen mustard, carmustine, camptothecin, hydroxycamptothecin, topotecan, irinotecan, capecitabine, gemcitabine, methotrexate, 5-fluorouracil, pemetrexed, cytarabine, apatinib, axitinib, cabozantinib, sorafenib, sunitinib, nivolumab, pembrolizumab, and ipilimumab.

11. A method for preventing, treating, or mitigating an intestinal disease, comprising administering to a subject a therapeutically effective amount of the microecological composition according to any one of claims 3-5, wherein
the intestinal disease is selected from:
intestinal diseases caused by any one, any two, any three, any four, any five, any six, any seven, or eight of *Pseudomonas aeruginosa, Shigella dysenteriae, Staphylococcus aureus, Escherichia coli, Salmonella paratyphi B, Yersinia enterocolitica, Vibrio parahaemolyticus,* and *Clostridioides difficile;*
oxidative damage in the intestinal tract;
diarrhea caused by an anti-tumor drug;
intestinal inflammation caused by an anti-tumor drug;
intestinal injury caused by an anti-tumor drug;
diarrhea caused by radiotherapy;
intestinal inflammation caused by radiotherapy; and
intestinal injury caused by radiotherapy.

12. The method according to claim 11, wherein the subject is selected from a human and a mouse.

13. The method according to claim 11, wherein the administration to the subject is selected from oral administration, intraperitoneal injection, and intragastric administration.

14. The method according to claim 11, wherein based on the total bacterial content in the probiotic composition, the therapeutically effective amount is 10⁶⁻¹² CFU per day.

15. The method according to claim 11, wherein the anti-tumor drug is selected from epirubicin, actinomycin D, doxorubicin, daunorubicin, paclitaxel, docetaxel, albumin-bound paclitaxel, cisplatin, carboplatin, nedaplatin, oxaliplatin, lobaplatin, cyclophosphamide, nitrogen mustard, carmustine, camptothecin, hydroxycamptothecin, topotecan, irinotecan, capecitabine, gemcitabine, methotrexate, 5-fluorouracil, pemetrexed, cytarabine, apatinib, axitinib, cabozantinib, sorafenib, sunitinib, nivolumab, pembrolizumab, and ipilimumab.
